(19) Europäisches Patentamt
European Patent Office
Office européen des brevets

(11) Publication number: **0 277 599**
**A2**

# (12) EUROPEAN PATENT APPLICATION

(21) Application number: **88101269.4**

(22) Date of filing: **28.01.88**

(51) Int. Cl.⁴: **C07D 473/34**, C07D 239/54, C07D 239/46, C07D 473/18, C07D 473/16, C07D 487/04, C07D 239/50, C07C 103/38, //C07C91/14,(C07D487/04, 249:00,239:00)

(30) Priority: **30.01.87 JP 18573/87**
**10.02.87 JP 27290/87**
**31.07.87 JP 190454/87**
**14.08.87 JP 201869/87**
**14.08.87 JP 201870/87**
**16.10.87 JP 259646/87**
**20.10.87 JP 262748/87**
**20.10.87 JP 262749/87**

(43) Date of publication of application:
**10.08.88 Bulletin 88/32**

(84) Designated Contracting States:
**DE FR GB IT**

(71) Applicant: **ASAHI GLASS COMPANY LTD.**
**No. 1-2, Marunouchi 2-chome**
**Chiyoda-ku, Tokyo(JP)**

(72) Inventor: **Morizawa, Yoshitomi**
**3-16-1, Sugita Isogo-ku**
**Yokohama-shi Kanagawa-ken(JP)**
Inventor: **Nakayama, Toshiaki**
**2-59-1, Tsurugamine Asahi-ku**
**Yokohama-shi Kanagawa-ken(JP)**
Inventor: **Yasuda, Arata**
**3-18-18, Jindaiji Kanagawa-ku**
**Yokohama-shi Kanagawa-ken(JP)**
Inventor: **Uchida, Keiichi**
**1-23-13, Kamikihon-cho Miyamae-ku**
**Kawasaki-shi Kanagawa-ken(JP)**

(74) Representative: **Wächtershäuser, Günter, Dr.**
**Tal 29**
**D-8000 München 2(DE)**

(54) **Fluorine containing cyclopentane derivatives and processes for their production.**

(57) A 1β-substituted-2-hydroxy(or unsubstituted)-3(or 4)-fluoro-4β-hydroxymethyl cyclopentane derivative having the formula:

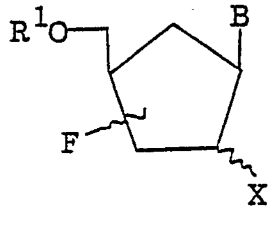

(I)

wherein B is a residue of a nucleic acid base, an azido group, an amino group or an amino group protected by a protecting group; $R^1$ is a hydrogen atom or a hydroxyl-protecting group; and X is a hydrogen atom or $-OR^2$ wherein $R^2$ is a hydrogen atom or a hydroxyl-protecting group, provided that the fluorine atom F is

EP 0 277 599 A2

present at the 3α-, 3β-or 4α-position.

# FLUORINE CONTAINING CYCLOPENTANE DERIVATIVES AND PROCESSES FOR THEIR PRODUCTION

The present invention relates to novel fluorine-containing cyclopentane derivatives and processes for their production.

Nucleosides having a fluorine-containing sugar are known as antitumor agents or antiviral agents. Specifically, for example, 3-deoxy-3-fluorofuranoside derivatives are known (Japanese Unexamined Patent publication No. 175498/1984, J.A. Wright et al, Carbohydrate Research, 18, 345-347 (1971)). However, in many cases, their activities are lower in vivo than in vitro.

The present inventors considered that the lower activities in vivo are attributable to chemical instability of the compounds in vivo and have prepared and studied carbocyclic nucleosides.

Carbocyclic nucleosides have a structure such that the oxygen atom of the ether moiety of the sugar is substituted by a carbon atom, and by this substitution, it is expected that the glycosidic linkage will be stabilized, whereby the chemical stability in vivo will be improved and the stability against an enzyme such as phosphoribosyl transferase will be improved. aristeromycine and neplanocin are known as carbocyclic nucleosides. However, heretofore, fluorine-containing carbocyclic nucleosides have not been known.

The present invention provides a 1β-substituted-2-hydroxy(or unsubstituted)-3(or 4)-fluoro-4β-hydroxymethyl cyclopentane derivative having the formula:

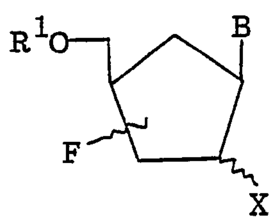

(I)

wherein B is a residue of a nucleic acid base, an azido group, an amino group or an amino group protected by a protecting group; $R^1$ is a hydrogen atom or a hydroxyl-protecting group; and X is a hydrogen atom or $-OR^2$ wherein $R^2$ is a hydrogen atom or a hydroxyl-protecting group, provided that the fluorine atom F is present at the 3α-, 3β-or 4α-position.

In the present invention, in the chemical structure, a bond extending upwards from a vertex of the pentagon (i.e. the cyclopentane ring) represents a β-configuration, and a bond extending downwards from a vertex of the pentagon represents an α-configuration. A bond represented by a wave line (∿∿) represents either α-or β-configuration. Thus, in the formula I, each of substituent B and $-CH_2OR^1$ is present at the β-position. For the purpose of the present invention, the position on the cyclopentane ring at which substituent B is bonded is designated as the 1-position, and likewise the position at which $-CH_2OR^1$ is bonded is designated as the 4-position. This designation corresponds to the positional designation of pentofuranose, and does not necessarily follow the standard nomenclature. However, the compounds in the Examples given hereinafter were named individually, and their names do not necessarily follow the above-mentioned positional designation. In the formula I, the fluorine atom F is located at the 3-or 4-position. When it is located at the 3-position, it takes the α-or β-configuration, and when it is located at the 4-position, it takes the α-configuration. X takes the α-or β-configuration at the 2-position.

A compound of the formula I wherein B is a residue of a nucleic acid base and which has no protecting group (i.e. $R^1$ is a hydrogen atom, and X is a hydrogen atom or a hydroxyl group) is a carbocyclic nucleoside for the purpose of the present invention. A compound of the formula I wherein B is a residue of a nucleic acid base and which has at least one protecting group, is a carbocyclic nucleoside protected by a protecting group, which can be converted to the above carbocyclic nucleoside by removing the protecting group therefrom. When B is other than the residue of a nucleic acid base, such a compound is useful as a precursor for the preparation of the above carbocyclic nucleoside. In particular, a compound of the formula I wherein B is an amino group can be converted to the one wherein B is the residue of a nucleic acid base. The compound of the formula I wherein B is an azido group is useful as a precursor for the compound of the formula I wherein B is an amino group. The azido group can readily be converted to an amino group. A compound of the formula I wherein B is an amino group protected by a protecting group is a useful compound for the fluorinating reaction or the reduction reaction as mentioned hereinafter in that the amino group is protected so as not to react during such a reaction. As the amino-protecting group, an acyl group or an alkyl group is suitable. Particularly preferred are a benzyl group, an acetyl group, a benzoyl group

and a pivaloyl group.

When $R^1$ or $R^2$ is a hydroxyl-protecting group, it may be a known hydroxyl-protecting group. For example, it may be a triorganosilyl group (the organic group may be alkyl, aryl or aralkyl), an acyl group or an aralkyl group. The aryl group or the aralkyl group may have a substituent such as an alkyl group or an alkoxy group on its aromatic nucleus. The three organic groups bonded to the silicon atom of the triorganosilyl group may be the same or different. Specifically, the hydroxyl-protecting group may be a trimethylsilyl group, a triethylsilyl group, a t-butyldimethylsilyl group, a phenyldimethylsilyl group, an acetyl group, a benzoyl group, a benzyl group, a trityl group, a dimethoxytrityl group or a monomethoxytrityl group. Further, in some cases, an alkyl group may be used as the protecting group. As such an alkyl group, a lower alkyl group, particularly a methyl group, is suitable.

In the present invention, the nucleic acid base may be a nucleic acid base or its derivative or a nucleic acid base analogue. The nucleic acid base or its derivative includes a prine or pyrimidine which may have a substituent. Its residue B includes a 9-purinyl group and 1-pyrimidinyl group, which may have a substituent. The substituent includes an amino group, an oxo group and a methyl group as well as other substituents such as a halogen atom, an alkyl group, a haloalkyl group, a halovinyl group, an alkoxy group, a hydroxyalkyl group, an alkylamino group, a dialkylamino group, an acylamino group, a mercapto group, an alkylthio group, a cycloalkyl group, an aryl group, an aryloxy group and an aralkyl group. Here, the halogen atom includes fluorine, chlorine, bromine and iodine. The same applies hereinafter, unless otherwise specified. Such a substituent may be attached at at least one of the 2-, 6-and 8-positions in the case of purines and at least one of the 2-, 4-and 5-positions in the case of pyrimidines. Further, an amine oxide is useful in which an oxygen atom is bonded to a nitrogen atom of the ring. Specific examples of such a substituted purine include, for example, adenine, guanine, hypoxanthine, xanthine, 2,6-diaminopurine, 6-halopurine, 2-halopurine, 2,6-dihalopurine, 6-alkylaminopurine, 6-acylaminopurine, adenine 1-oxide and adenine 7-oxide. Likewise, specific examples of the substituted pyrimidine include, for example, uracil, cytosine, thymine, 5-halouracil, 5-halomethyluracil, 5-halothymine, 5-halomethylthymine and 5-$\beta$-bromovinylthymine. A preferred nucleic acid base or its derivative is a 2-and/or 6-substituted purine or a 2,4-or 2,4,5-substituted pyrimidine. Particularly preferred are adenine, guanine, hypoxanthine, xanthine, 2-haloadenine, $N^6$-substituted adenine, 2,6-diaminopurine, 6-halopurine, 2,6-dihalopurine, uracil, cytosine, thymine and 5-halouracil.

In the present invention, the nucleic acid base homologue means a heterocyclic compound having a ring corresponding to a purine or pyrimidine, or its derivative. Its residue B is meant for a residue having a bond at the position corresponding to the 9-position of the purine or the 1-position of the pyrimidine. As such a heterocyclic compound, the one commonly known as an analogue of a nucleic acid base is preferred. Specifically, a heterocyclic compound obtained by replacing at least one of nitrogen atoms in the purine or pyrimidine ring by a carbon atom or by a heteroatom other than a nitrogen atom, or by replacing at least one of carbon atoms in the ring by a nitrogen atom or by other heteroatom, or by conducting both of such replacements, is preferred. More preferred are a heterocyclic compound obtained by replacing one nitrogen atom (particularly a nitrogen atom at the 1-, 3-or 7-position) of the purine ring by a carbon atom or by an oxygen atom, a heterocyclic compound obtained by replacing one carbon atom (particularly a carbon atom at the 2-, 5-or 8-position) in the purine ring by a nitrogen atom and a heterocyclic compound obtained by conducting both of such replacements (i.e. by replacing one nitrogen atom by a carbon atom or by an oxygen atom and by replacing one carbon atom by a nitrogen atom). Likewise, with respect to the pyrimidine, a heterocyclic compound obtained by replacing the nitrogen atom at the 3-position by a carbon atom or by replacing the carbon atom at the 5-or 6-position by a nitrogen atom, is preferred. The derivative of such a heterocyclic comound is a compound having the same substituent as mentioned above introduced. Particularly preferred as the substituent is an amino group, an oxo group, a halogen atom or a methyl group. The specific compound includes, for example,
5-amino-3,6-dihydro-7H-1,2,3-triazine[4,5-d]pyrimidin-7-one (the first chemical structure in the following list of structures) which is a guanine analogue. Specific examples of the residue of such a nucleic acid base analogue are shown below. However, the residue of such an analogue is not limited to such specific examples. In the bracket below each chemical formula, the corresponding nucleic acid base is indicated.

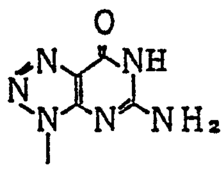

(guanine)

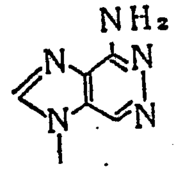

(adenine)

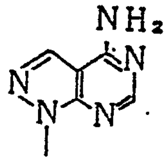

(adenine)

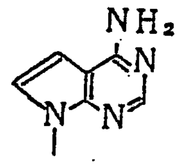

(adenine)

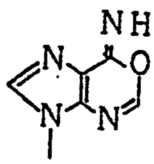

(adenine)

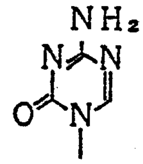

(cytosine)

The compound of the formula I of the present invention can be prepared by various processes. The basic method is the fluorination at the 3-position. Namely, a fluorine atom is introduced to the 3-position by fluorination of a compound having a hydroxyl group at the 3-position or by fluorination of a 2,3-epoxy compound. A compound having a fluorine atom at the 4-position is produced simultaneously during the fluorination of the compound having a hydroxyl group at the 3-position. By the fluorination of a compound having a hydroxyl group at the 3-position or a 2,3-epoxy compound, the fluorine atom is usually introduced to the 3-position to take a steric position where no oxygen atom exists. For example, from a 3$\beta$-hydroxy compound, a 3$\alpha$-fluoro compound will be obtained. Likewise, from a 2$\alpha$,3$\alpha$-epoxy compound, a 3$\beta$-fluoro compound will be obtained, and from a 2$\beta$,3$\beta$-epoxy compound, a 3$\alpha$-fluoro compound will be produced. In the fluorination of the latter two epoxy compounds, a hydroxyl group (or its derivative group) will be formed at the 2-position, and its steric configuration will be trans to the fluorine atom at the 3-position i.e. the same configuration as the oxygen atom of the epoxy group in the starting epoxy compound For instance, from a 2$\beta$,3$\beta$-epoxy compound, a 2$\beta$-hydroxy-3$\alpha$-fluoro compound will be formed, and from a 2$\alpha$,3$\alpha$-epoxy compound, a 2$\alpha$-hydroxy-3$\beta$-fluoro compound will be formed.

A compound of the formula I wherein X is a hydrogen atom may preferably be prepared from a compound having a fluorine atom at the 3-or 4-position and a hydroxyl group at the 2-position (this starting material itself is a compound of the formula I) by converting the hydroxyl group at the 2-position to a hydrogen atom. The reaction to convert the hydroxyl group to a hydrogen atom is basically a reduction reaction, and this reaction will be hereinafter referred to as a reduction reaction. Otherwise, the same compound can be produced likewise from a derivative having a bromine atom at the 2-position by converting the bromine atom to a hydrogen atom.

In the above-mentioned fluorination reaction, if a hydroxyl group is present at a position other than the 3-position, such a position is likely to be fluorinated. Accordingly, the hydroxyl group at a position which is not desired to be fluorinated, is preferably protected during the fluorination. Likewise, in the above-mentioned reduction reaction, a hydroxyl group at a position which should not be reduced is preferably protected. On the other hand, when a compound of the formula I wherein B is a residue of a nucleic acid base is fluorinated, it is likely that the residue is attacked by the fluorinating agent and a byproduct will be formed. Accordingly, in the fluorination reaction stage, the compound to be fluorinated preferably has no residue of a nucleic acid base at the 1-position. However, this is not an essential requirement. For example, it is possible to fluorinate a 2,3-epoxy compound having a residue of a nucleic acid base at the 1-position to obtain a corresponding 3-fluoro compound. Further, the residue of nucleic acid base may be present at the 1-position during the reaction for reducing the hydroxyl group at the 2-position. If necessary, a functional

group such as an amino group in the residue of a nucleic acid base can suitably be protected during the fluorination or reduction of such a compound having the residue of a nucleic acid base.

Various conventional methods may be employed to convert an amino group at the 1-position of the compound of the formula I to a residue of a nucleic acid base. For example, a method disclosed in literatures (S Daluge et al, J. Org. Chem., 43, 2311 (1978), Y.F. Shealy et al, J. Am. Chem. Soc., 91, 3075 (1969), Y.F. Shealy et al, J. Heterocyclic Chem., 13, 1015 (1976)) may be employed. According to this method, for example, in the case of adenosine, 5-amino-4,6-dichloropyrimidine and a compound of the formula I wherein each of $R^1$ and $R^2$ is a hydrogen atom and B is an amino group are reacted to obtain a diaminopyrimidine derivative, which is then reacted with ethyl orthoformate to form a purine nucleus. The chlorine atom at the 6-position of the purine ring can be treated with ammonia for conversion to adenosine. Further, a residue of a nucleic acid base at the 1-position can be converted to other residue of a nucleic acid base.

The following Processes A to C are preferred for the production of derivatives of the formula I by means of the above basic methods. The first step in each of Processes A and B is fluorination, and the first step in Process C is reduction. A product having no residue of a nucleic acid base at the 1β-position is subjected to a reaction for introducing a residue of a nucleic acid base. The second step in each of these processes is the reaction for introducing a residue of a nucleic acid base. In the following formulas, B′ represents the residue of a nucleic acid base. All of the products of the formulas I-1 to I-6 are compounds falling within the scope of the formula I.

Process A

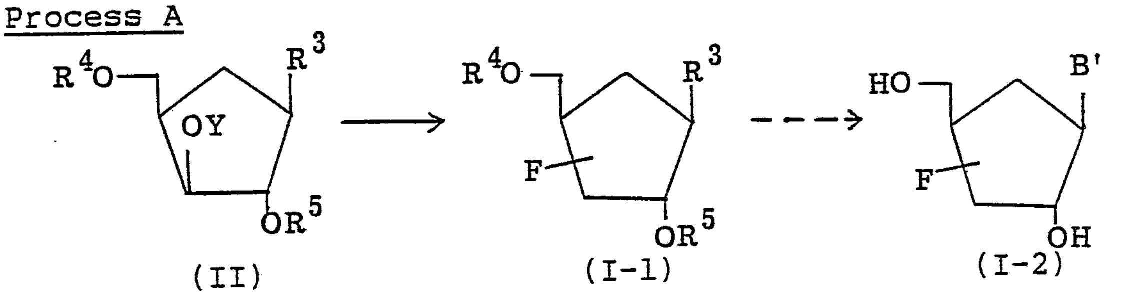

(II) (I-1) (I-2)

Process B

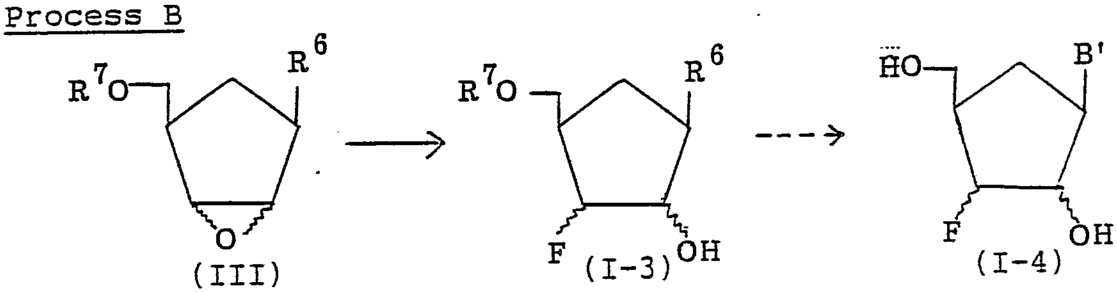

(III) (I-3) (I-4)

Process C

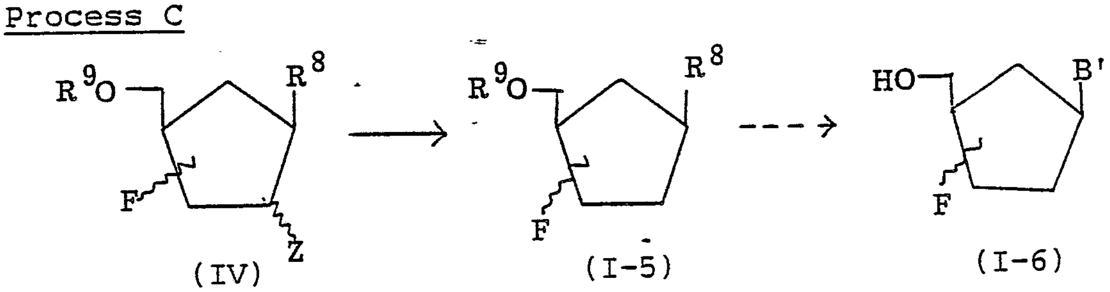

(IV) (I-5) (I-6)

Process A

The compound of the formula II is a 1β-substituted-2α,3β-dihydroxy-4-hydroxymethyl cyclopentane derivative (hereinafter referred to also as a cyclopentane triol derivative). $R^3$ at the 1-position is an azido group or an amino group protected by a protecting group. Particularly preferred are an azido group and an amino group protected by an acyl group. $R^4$ and $R^5$ are hydroxyl-protecting groups. These protecting groups may be those mentioned above.

Y may be a hydrogen atom. In such a case, however, the removal of the hydroxyl group bonded at the 3-position and the subsequent fluorination at the 3-or 4-position are not necessarily easy, and it will be important to select the fluorinating agent properly. Namely, as will be described hereinafter, when a metal fluoride or a quaternary ammonium fluoride is used as the fluorinating agent, if Y is a hydrogen atom, the removal of -OY tends to hardly adequately proceed, and it is advisable to convert Y to an active group (i.e. an active group capable of facilitating the removal of -OY) such as a sulfonyl group, before the removal. When the fluorinating agent is an amino sulfur fluoride type fluorinating agent, the removal can be made even if Y is a hydrogen atom. However, Y may be converted to an active group for removal such as a triorganosilyl group, so that the removal can further be facilitated.

When the cyclopentanetriol derivative of the formula II is fluorinated with a fluorinating agent, -OY is removed and a fluorine atom is introduced at the 3-or 4-position. The fluorine atom introduced at the 3-position takes an α-configuration. The product is usually a mixture of a 3-fluoro derivative and a 4-fluoro derivative. From this mixture, the 3-fluoro derivative and the 4-fluoro derivative can readily be separated. Under a usual fluorination reaction condition, the production ratio of the 3-fluoro derivative to the 4-fluoro derivative is usually within a range of from 1:9 to 9:1. Depending upon the particular purpose, it is possible to control the reaction condition to increase the production ratio of one of the derivatives. The 3-fluoro derivative and the 4-fluoro derivative can be separated based on the difference in their physical or chemical properties. For example, they can readily be separated by e.g. column chromatography. The separation of the 3-fluoro derivative and the 4-fluoro derivative may be conducted at an optional stage after the fluorination. For example, the separation can be conducted at the stage of the fluorination prior to the introduction of a residue of a nucleic acid base, at a stage after the removal of the protecting group, at a stage during the introduction of a nucleic acid base or at a stage after the introduction of a nucleic acid base.

When the fluorination is conducted after activating the hydroxyl group at the 3-position, the hydroxyl group at the 3-position is converted to an active group (Y). For example, as such an active group, a sulfonyl group such as a methanesulfonyl group, a trifluoromethanesulfonyl group, p-toluenesulfonyl group or an imidazolylsulfonyl group and an acetyl group may be mentioned. A trifluoromethanesulfonyl group has a particularly high active property and can be used as a preferred active group. In this case, a known fluorinating agent such as a fluoride of a metal such as potassium or a fluoride of a quaternary ammonium may be used as the fluorinating agent. Particularly preferred is a tetraalkyl(or aralkyl)ammonium fluoride. As the alkyl group, a lower alkyl group is suitable, and as the aralkyl group, a benzyl group is suitable. The four alkyl or aralkyl groups may be the same or different, and they may be a mixture of alkyl and aralkyl groups. It is preferred to use tetrabutylammonium fluoride.

The fluorination is usually conducted in an inert solvent such as tetrahydrofuran or acetonitrile at a temperature of not higher than a few tens degree in centigrade. It is particularly preferred to conduct the reaction at a temperature of from -70°C to room temperature.

It is also possible to conduct the fluorination while maintaining the hydroxyl group at the 3-position as it is or after converting it to a trimethylsilyl group or a triethylsilyl group as an active group (Y). As the fluorinating agent in this case, an amino sulfur fluoride type fluorinating agent is used. This fluorinating agent is a compound of the following formula V or VI having a >N-SF group.

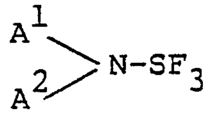

(V)

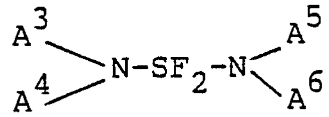

(VI)

wherein each of $A^1$, $A^2$, $A^3$, $A^4$, $A^5$ and $A^6$ which may be the same or different is a monovalent organic group, or $A^1$ and $A^2$, $A^3$ and $A^4$, or $A^5$ and $A^6$ form together with the adjacent nitrogen atom a heterocyclic ring.

In the above formulas V and VI each of $A^1$ to $A^6$ is preferably an alkyl group, a cycloalkyl group, an aryl group or an aralkyl group. Or, it is preferred that $A^1$ and $A^2$, $A^3$ and $A^4$, or $A^5$ and $A^6$ form together with the adjacent nitrogen atom a 5-7 membered nitrogen-containing heterocyclic group, particularly a 5-6 membered non-aromatic heterocyclic group having one nitrogen atom or one nitrogen atom and one oxygen atom. Specifically, the compound of the formula V includes dimethylaminosulfur trifluoride, diethylaminosulfur trifluoride, diisopropylaminosulfur trifluoride, piperidinosulfur trifluoride and morpholinosulfur trifluoride. Likewise, the compound of the formula VI includes bis(dimethylamino)sulfur difluoride, bis(diethylamino)-sulfur difluoride, dimethylaminodiethylaminosulfur difluoride and diethylaminopiperidinosulfur difluoride. Particularly preferred fluorinating agents are those represented by the above formula V such as diethylaminosulfur trifluoride and piperidinosulfur trifluoride.

In this case, the fluorination reaction is usually conducted in an inert solvent. As such a solvent, an aliphatic hydrocarbon solvent such as hexane or pentane, an aromatic hydrocarbon solvent such as benzene or toluene, or a halogenated hydrocarbon solvent such as 1,1,2-trichloro-1,2,2-trifluoroethane, dichloromethane, chloroform or carbon tetrachloride, may suitably be employed. However, the solvent is not restricted to such specific examples. Preferably, 1,1,2-trichloro-1,2,2-trifluoroethane or dichloromethane is employed.

The fluorinating agent is used usually in an amount of from 1 to 10 equivalent relative to the substrate, although there is no particular restriction as to the amount of the fluorinating agent to the substrate. The reaction temperature is usually within a range of from about -78°C to the refluxing temperature of the solvent.

The cyclopentanepolyol derivative of the formula II is required to be prepared stereospecifically. Further, hydroxyl groups other than the hydroxyl group at the 3-position must selectively be protected so that they will not be affected by the fluorination reaction. For these reasons, the cyclopentanepolyol derivative of the formula II should preferably be prepared by the following route. The compound of the formula 1 is a compound known in literatures (S.B. Jorgnsen et at, Acta Chemical Scandinavica, 20, 2192-2164 (1966), A. Maercker et al, Chem. Ber., 106, 773-797 (1973), H. Paulsen et al, Chem. Ber., 114, 346-358 (1981).

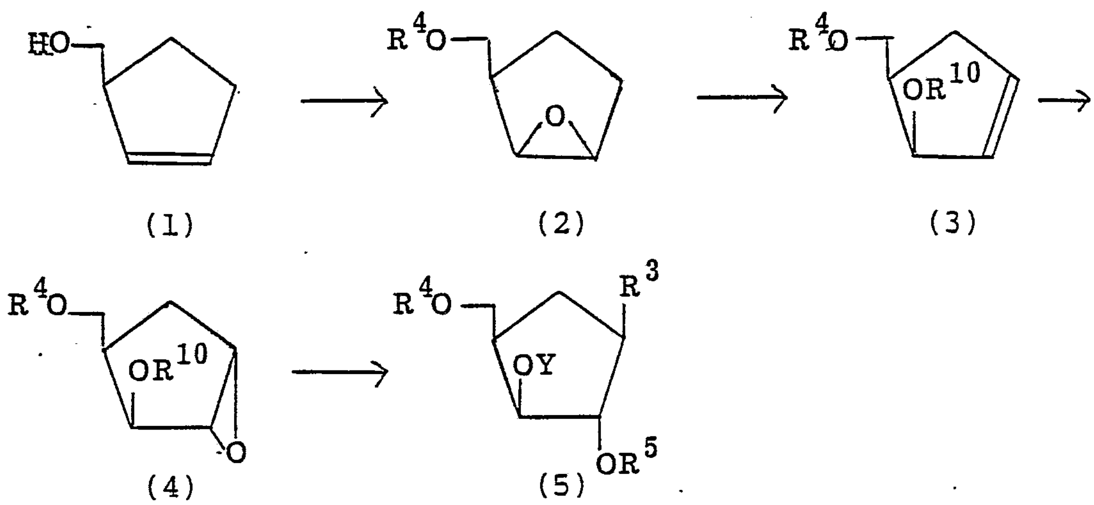

(1) (2) (3) (4) (5)

The compound of the formula 1 is oxidized to form an epoxide (a compound of the formula 2 wherein $R^4$ is a hydrogen atom). As the oxidizing agent, an organic peroxy carboxylic acid such as performic acid, peracetic acid or m-chloroperbenzoic acid, or t-butylhydroperoxide or other peroxide may be used. In order to conduct the epoxidation stereospecifically, it is particularly preferred to use a metal catalyst such as molybdenum hexacarbonyl/t-butylhydroperoxide or vanadylacetyl acetonate/t-butylhydroperoxide. Further, in order to obtain an asymmetric epoxide, it is also possible to employ (+)-dimethyl or diisopropyl tartarate-titanium tetraisopropoxide/t-butylhydroperoxide.

Then, the hydroxyl group is protected. As the protecting group, a methyl group, a benzyl group, a trityl group or a t-butyldimethylsilyl group is preferred. Particularly preferred is a benzyl group.

Then, the epoxide of the formula 2 is subjected to ring opening in the presence of a reducing agent to

form a cyclopentene alcohol (a compound of the formula 3 wherein $R^{10}$ is a hydrogen atom). As the ring opening agent for the epoxide, an organic selenium compound such as diphenyldiselenide is used. The organoseleno group thus introduced can readily be converted to a cyclopentene alcohol (a compound of the formula 3 wherein $R^{10}$ is a hydrogen atom) by an oxidizing agent such as a 30% hydrogen peroxide solution.

Then, the hydroxyl group at the 3-position is protected. As the protecting group, a trimethylsilyl group, a triethylsilyl group, a t-butyldimethylsilyl group or a t-butyldiphenylsilyl group is preferably employed. For the subsequent step, it is particularly preferred to employ a sterically bulky t-butyldimethylsilyl or t-butyldiphenylsilyl group.

Then, the 1,2-olefinic double bond is epoxidized. As the oxidizing agent, a peroxycarboxylic acid such as peracetic acid or m-chloroperbenzoic acid is suitable. By virtue of the steric effect of the hydroxyl-protecting group at the 3-position employed for the compound of the formula 3, the resulting epoxide will be a 1$\alpha$,2$\alpha$-epoxycyclopentane derivative.

Then, the formed epoxide is converted to an amino alcohol. For this ring-opening reaction of the epoxide, sodium azide is used. The azido group is introduced to the 1-position, while the hydroxyl group at the 2-position is protected. As the protecting group, a methyl group or a benzyl group may be used. It is preferred to employ a benzyl group. Then, the hydroxyl-protecting group at the 3-position is removed. For the removal of the protecting group, a tetraalkylammonium fluoride, preferably tetrabutylammonium fluoride, or cesium fluoride is used, to obtain an azido cyclopentanepolyol derivative (when $R^3$ is azido and Y is a hydrogen atom in the formula 5, the formula 5 agrees to the formula II).

Otherwise, the epoxide can likewise be converted to an amino alcohol by reacting it with acetonitrile in the presence of a boron trifluoride-ether complex, followed by acetylation.

As mentioned above, by fluorinating a cyclopentane polyol derivative of the formula II with a fluorinating agent, the removal of the OY group and the introduction of the fluorine atom take place. When a fluorine atom is introduced at the 3-position, the fluorine atom will be bonded to a side sterically opposite to the OY group. The reason for the introduction of a fluorine atom at the 4-position may be such that the 4-position is activated by the removable property of the OY group at the 3-position.

Then, the removal of the hydroxyl-protecting group or the reduction of the azido group will be conducted. It is particularly preferred to conduct both reactions simultaneously. This can be done by adding hydrogen in the presence of a palladium-carbon catalyst. The amino diol thus obtained (a compound of the formula I-1 wherein each of $R^4$ and $R^5$ is a hydrogen atom and $R^3$ is an amino group) is then subjected to the formation of a residue of a nucleic acid base as described hereinbefore.

## Process B

Process B is a method for fluorinating the 2,3-epoxy compound of the formula III. $R^6$ is an azido group, an amino group protected by a protecting group or a residue of a nucleic acid base as mentioned above. When $R^6$ is a residue of a nucleic acid base and the residue has a highly reactive functional group such as an amino group, such a functional group is preferably protected by a protecting group. $R^7$ is a hydroxyl-protecting group, which is preferably as described above. As the fluorinating agent, a hydrogen fluoride type fluorinating agent is preferred to the above described amino sulfur fluoride type fluorinating agent. As such a hydrogen fluoride type fluorinating agent, an anhydrous hydrogen fluoride-organic base system may be employed, or anhydrous hydrogen fluoride may be used alone. As the organic base, a primary alkyl amine, a secondary alkyl amine or tertiary alkyl amine may be mentioned, wherein the alkyl group may preferably be straight chained or branched with from 1 to 10 carbon atoms. It is also possible to employ a cyclic amine such as pyrrolidine, piperazine or morpholine or an organic amine such as aniline, N,N-dimethylaniline, m-phenylene diamine, pyridine, melamine, 1,3,5-triazine, cyanuric acid, cyanuric chloride or 2,4,6-triphenyl-1,3,5-triazine. The equivalent ratio of the organic base to anhydrous hydrogen fluoride may be optionally selected within a range of from 0 to 100%, preferably from 1 to 50%.

As the solvent, an aliphatic hydrocarbon solvent, an aromatic hydrocarbon solvent or a halogenated solvent such as dichloromethane, chloroform, carbon tetrachloride or 1,1,2-trichloro-1,2,2-trifluoroethane may be used. It is preferred to employ a halogenated solvent.

The reaction temperature may be from -100°C to the refluxing temperature of the solvent. It is preferably from 0°C to room temperature.

The compound of the formula III is a known compound (S.Daluge, R. Vince, J. Org. Chem., 43, 2311 (1978), which may be prepared stereospecifically. It may be prepared by epoxidizing a known carbocyclic type nucleoside (e.g. the above-mentioned aristeromycine: J.G. Moffatt et al, J. Am. Chem. Soc., 95, 4025

(1975)).

When $R^6$ in the formula III is other than the residue of a nucleic acid base, it is preferred to introduce a residue of a nucleic acid base after the fluorination. In such a case, $R^6$ is preferably an amino group protected by an acyl group such as an acetamido group, or an azido group. After the fluorination, this $R^6$ is converted to an amino group, which is then converted to a residue of a nucleic acid base in accordance with the method described hereinbefore. The removal of the hydroxyl-protecting group may be conducted simultaneously or subsequent to the conversion of $R^6$ to an amino group.

Process C

In the formula IV $R^8$ is an azido group, an amino group protected by a protecting group or a residue of a nucleic acid base. When $R^8$ is a residue of a nucleic acid base, the functional group in the residue such as an amino group is preferably protected by a protecting group. $R^9$ is a hydroxyl-protecting group, which may suitably be as described above. Z is a hydroxyl group, an activated hydroxyl group as mentioned hereinafter or a bromine atom.

Z may be a hydroxyl group. However, in such a case, a relatively strong reducing agent will be required, and the $-OR^9$ group may also be thereby affected. Therefore, Z is preferably a readily reducible group. For this purpose, it is preferred that a reacting agent is reacted to the hydroxyl group to convert Z to an activated hydroxyl group before the reduction. As such a reacting agent, N,N'-thiocarbonyl diimidazole, phenylchlorothionocarbonate or N,N'-dimethylbenzamide-phosgene-hydrogensulfide (D.H.R. Barton et al, J.C.S. Perkin (1975) 1574) may be employed. As the reducing agent, tributyltinhydride or triphenyltinhydride may be employed. It is also possible to convert the hydroxyl group to a m-trifluoromethylbenzoyl and then selectively removing the hydroxyl group by irradiation with light (T. Matumura et al, J. Am. Chem. Soc., (1986) 108, 3115). This reduction reaction is conducted usually in an inert solvent such as benzene or toluene within a temperature range of from 0°C to the refluxing temperature of the solvent, preferably within a range of from room temperature to 120°C. As a reaction initiator, it is preferred to employ N,N-azobisisobutyronitrile or benzoyl peroxide.

The compound of the formula IV corresponds to a compound of the formula I of the present invention having a hydroxyl group at the 2-position. Accordingly, this starting material may suitable be the one produced by the above-mentioned Process A or B. When $R^8$ is not a residue of a nucleic acid base, it is preferred to introduce a residue of a nucleic acid base at the 1-position after the reduction. In such a case, $R^8$ is preferably an amino group protected by an acyl group such as an acetamido group, or an azido group. After the reduction, $R^8$ is converted to an amino group, and then converted to a residue of a nucleic acid base in accordance with the above-mentioned method. The removal of the hydroxyl group may be conducted simultaneously or subsequent to the reaction to convert $R^8$ to an amino group.

Now, the present invention will be described in further detail with reference to Preparation Examples and Working Examples. However, it should be understood that the present invention is by no means restricted by these specific Examples. In these Examples, the positional numbers for the substituents were determined for indivisual compounds, and they are in some cases different from the positional numbers in the foregoing description (namely, in some cases, the above-mentioned 1-position is designated as the 4-position, and the above-mentioned 4-position is designated as the 1-position).

The following Preparation Examples describe the preparation of the above-mentioned compounds 1 to 5 i.e. the derivatives of the formula II. $R^3$ in the compound 5 is an azido group.

Example 1 shows the fluorination at the 3-and 4-positions in accordance with the above-mentioned Process A and the subsequent conversion of the azido group at the 1-position to an amino group. It further includes the conversion of the amino group to an adenyl group.

Examples 2 to 10 illustrate the conversion of the amino group in the derivative obtained in Example 1 wherein the 1-position is an amino group, to various residues of nucleic acid bases and the conversion of one residue of a nucleic acid base to another.

Example 11 illustartes the fluorination of a derivative of the formula III wherein $R^6$ is an acetamido group and the epoxy group takes a $\beta$-configuration, in accordance with Process B, and Example 12 illustrates the conversion of the acetamido group of the product to an amino group and the further conversion to an adenyl group.

Examples 13 to 20 illustrate the conversion of the amino group of the derivative obtained in Example 12 wherein the 1-position is the amino group, two various residues of nucleic acid bases and the conversion of one residue of a nucleic acid base to another.

Example 21 illustrates the fluorination of a derivative of the formula III wherein $R^6$ is an adenyl group

and the epoxy group takes an α-configuration, in accordance with Process B.

Example 22 illustrates the reduction of the hydroxyl group at the 2-position of the derivative obtained in Example 1 wherein $R^8$ is an adenyl group and the fluorine atom takes a 3α-configuration, in accordance with Process C.

Example 23 illustrates a case wherein a fluorine atom is introduced at the 3-position and a bromine atom is introduced at the 2-position using a known compound as the starting material (the 1-position is an acetamido group), and then the bromine atom at the 2-position was converted to a hydrogen atom.

Example 24 illustrates a case wherein the hydroxyl group at the 2-position is reduced by using the product of Example 11 as the starting material wherein the 1-position is an acetamido group, in accordance with Process C. The product thus obtained is the same as the product of Example 23.

Example 25 illustrates the conversion of the acetamido group at the 1-position of the product of Example 23 or the product of Example 24 which is the same compound as the product of Example 23.

Examples 26 to 30 illustrate the conversion of the amino group of the product of Example 25 to various residues of nucleic acid bases.

PREPARATION EXAMPLE 1

(1) <u>Preparation of 2β,3β-epoxycyclopentene-1-β-methanol (a compound of the formula 2 wherein $R^4$ is a hydrogen atom)</u>

A solution in benzene (20 ml) of 2.0 g (20 mmol) of 2-cyclopentene methanol was added to a suspension in benzene (80 ml) of 0.16 g (0.6 mmol) of molybdenum hexacarbonyl and 3.0 g (30 mmol) of t-butylhydroperoxide, and the mixture was refluxed for 1.5 hours under heating. The reaction mixture was cooled, then treated with a saturated sodium sulfite aqueous solution and extracted with ethyl acetate. The organic layer was dried over anhydrous magnesium sulfate, then concentrated and purified by column chromatography to obtain 1.6 g (yield: 70%) of an epoxy alcohol.

$^1$HNMR ($CDCl_3$): δ0.8-1.4(m, 1H), 1.4-2.5(m, 5H), 3.56(brs, 2H), 3.78(d, J=6.5hz, 2H)

(2) <u>Preparation of 2β,3β-epoxy-1β-cyclopentanemethyl benzyl ether (a compound of the formula 2 wherein $R^4$ is a benzyl group)</u>

5.7 g (0.13 mol) of sodium hydride (55%) was suspended in tetrahydrofuran (90 ml), and cooled to 0°C. Then, a solution in tetrahydrofuran (40 ml) of 12.32 g (0.11 mol) of the epoxy alcohol obtained in Preparation Example (1), was added thereto. The mixture was stirred at room temperature for 30 minutes, and then 25.9 g (0.15 mol) of benzyl bromide was added. The mixture was reacted for 30 minutes under reflux. The reaction mixture was treated in a usual manner to obtain 21.8 g (yield: 99%) of benzyl ether.

$^1$HNMR ($CDCl_3$): δ0.8-1.5(m, 5H), 2.4-2.7(m, 4H), 3.62(s, 2H), 7.44(s, 5H)

(3) <u>Preparation of 2β-hydroxy-3-cyclopentene-1-β-methyl benzyl ether (a compound of the formula 3 wherein $R^4$ is a benzyl group and $R^{19}$ is a hydrogen atom)</u>

33.3 g (0.11 mol) of diphenyl diselenide was suspended in ethanol (131 ml) and cooled to 0°C. Then, 0.07 g (0.21 mol) of sodium borohydride was gradually added thereto. The mixture was stirred at room temperature for 30 minutes, and a solution in ethanol (15.6 ml) of 21.7 g (0.11 mol) of the epoxide obtained in Preparation Example (2) was dropwise added over a period of 45 minutes. The mixture was refluxed for 1 hour under heating, and then cooled, and 114.5 ml of a 30% hydrogen peroxide was dropwise added. The reaction temperature was controlled at a level not exceeding 15°C. After the completion of the reaction, the reaction mixture was treated in a usual manner to obtain 6.6 g of the above identified product.

$^1$HNMR ($CDCl_3$): δ2.0-2.7(m, 4H), 3.6-3.8(m, 2H), 4.60(s, 2H), 4.8-5.0(m, 1H), 5.8-6.2(m, 2H), 7.44(s, 5H)

(4) Preparation of 2β-t-butyldimethylsiloxy-3-cyclopentene-1β-methyl benzyl ether (a compound of the formula 3 wherein $R^4$ is a benzyl group and $R^{10}$ is a t-butyldimethylsilyl group)

To 6.58 g (32.3 mmol) of the alcohol obtained in Preparation Example (3), 5.8 g (38.8 mmol) of chloro-t-butyldimethylsilane was added for silylation. The reaction was conducted at 40°C for 1.5 hours. Then, ice water was added thereto, and the reaction mixture was extracted with hexane. The organic layer was dried and concentrated, and then purified by column chromatography to obtain 9.82 g (yield: 95%) of a silyl ether.

$^1$HNMR ($CDCl_3$): δ0.18(s, 6H), 1.41(s, 9H), 2.3-2.7(m, 3H), 3.5-4.0(m, 2H), 5.14(d, J=1.8Hz, 2H), 4.9-5.0-(m, 1H), 5.8-6.2(m, 2H), 7.50(s, 5H)

(5) Preparation of 2β-t-butyldimethylsiloxy-3α,4α-epoxycyclopentane-1β-methylbenzyl ether (a compound of the formula 4 wherein $R^4$ is a benzyl group and $R^{19}$ is a t-butyldimethylsilyl group)

9.10 g (28.5 mmol) of the product obtained in Preparation Example (4) was dissolved in dichloromethane (20 ml), and a suspension in dichloromethane of 6.22 g (34.2 mmol) of m-chloroperbenzoic acid was added thereto. The mixture was reacted for 2 hours, and then treated with a saturated sodium hydrogensulfite solution. Then, the mixture was purified by column chromatography to obtain 7.6 g (yield: 80%) of the above identified compound.

$^1$H NMR ($CDCl_3$): δ0.19(s, 3H), 0.22(s, 3H), 1.00(s, 9H), 1.3-1.8(m, 2H), 2.0-2.3(m, 2H), 3.4-3.8(m, 3H), 4.46(dis-tortod d, J=4.0Hz, 1H), 4.62(d, J=4.3Hz, 2H), 7.55(s, 5H)

(6) Preparation of 4β-azido-3α-hydroxy-2β-t-butyl-dimethylsiloxycyclopentane-1β-methyl benzyl ether (a compound of the formula 5 wherein $R^3$ is an azido group, $R^4$ is a benzyl group, $R^5$ is a hydrogen atom and Y is a t-butyldimethylsilyl group)

5.89 g (17.6 mmol) of the epoxide obtained in Preparation Example (5) was dissolved in water (20 ml) and 2-methoxyethanol (60 ml). Then, 1.26 g of ammonium chloride and 6.08 g of sodium azide were added thereto, and the mixture was reacted at 75°C for 18 hours. After the completion of the reaction, the solvent was distilled off, and the aqueous sodium chloride solution was added. And the mixture was extracted with ethyl ether. The extract was purified by column chromatography to obtain 3.52 g of the above identified purified product and 2.32 g of the starting material.

$^1$H-NMR ($CDCl_3$): δ0.04(s, 3H), 0.08(s, 3H), 0.98(s, 9H), 1.6-2.4(m, 4H), 3.3-3.8(m, 3H), 3.9-4.1(m, 2H), 4.5-4.6(m, 2H), 7.3-7.5(m, 5H)

(7) Preparation of 4β-azido-3α-benzyloxy-2β-t-butyldimethylsiloxycyclopentane-1β-methyl benzyl ether (a compound of the formula 5 wherein $R^3$ is an azido group, each of $R^4$ and $R^5$ is a benzyl group, and Y is a t-butyldimethylsilane group)

3.52 g (9.3 mmol) of the alcohol obtained in Preparation Example (6) was added to a suspension in tetrahydrofuran (20 ml) of 0.49 g (11.2 mmol) of sodium hydride. The mixture was stirred at room temperature for 30 minutes. Then, 2.2 g (13.0 mmol) of benzyl bromide was added thereto, and the mixture was refluxed for 1 hour under heating. The reaction mixture was treated in a usual manner and purified by column chromatography to obtain 4.0 g (yield: 93%) of the above identified purified product.

$^1$HNMR ($CDCl_3$): δ0.00(s, 6H), 0.84(s, 9H), 1.5-2.6(m, 3H), 3.3-3.8(m, 4H), 4.0-4.2(m, 1H), 4.4-4.6(m, 4H), 7.40(s, 10H)

(8) Preparation of 4β-azido-3α-benzyloxy-2β-hydroxycyclopentane-1β-methylbenzyl ether (a compound of the formula 5 wherein $R^3$ is an azido group, each of $R^4$ and $R^5$ is a benzyl group, and Y is a hydrogen atom)

4.05 g (8.65 mmol) of the silyl ether obtained in Preparation Example (7) was dissolved in tetrahydrofuran (15 ml). Then, a tetrahydrofuran solution of tetrabutylammonium fluoride (factor=1) (26 ml, 26 mmol) was added thereto over a period of 40 minutes, and the mixture was stirred at room temperature

for 2 hours. The solvent was distilled off, and a saturated ammonium chloride solution was added to the reaction mixture. The mixture was extracted with chloroform. The extract was purified by column chromatography to obtain 2.58 g (yield: 85%) of the above identified compound.

$^1$H-NMR ($CDCl_3$): δ1.6-2.6(m, 3H), 2.9-3.1(m, 1H), 3.6-3.9(m, 4H), 4.1-4.4(m, 1H), 4.56(s, 2H), 4.72(s, 2H), 7.44(s, 1H)

EXAMPLE 1

1.84 g (5.22 mmol) of the alcohol obtained in Preparation Example (8) was dissolved in dichloromethane (15 ml), and 4.2 ml (52 mmol) of pyridine and 2.0 ml (16.0 mmol) of chlorotrimethylsilane were added thereto. The mixture was stirred at room temperature for 30 minutes, and then treated in a usual manner. The crude product thus obtained was dissolved in dichloromethane (12 ml), and 1.0 ml (7.8 mmol) of piperidinoaminosulfur trifluoride was added thereto. The mixture was reacted at 0°C for 1 hour. Then, triethylamine (1.2 ml) was added thereto, and the mixture was treated with a saturated potassium carbonate aqueous solution. The mixture was purified by column chromatography (silica gel, hexene/ethyl acetate = 20/1) to obtain 0.30 g of a 3-fluoro derivative (10) and 0.35 g of a 4-fluoro derivative (11).

The 3-fluoro derivative (10) is 4β-azido-3α-benzyloxy-2α-fluoro-cyclopentane-1β-methylbenzyl ether, and the 4-fluoro derivative (11) is 4β-azido-3α-benzyloxy-1α-fluoro-cyclopentane-1β-methylbenzyl ether.

The spectrum data of the 3-fluoro derivative (10) are as follows:

$^{19}$F-NMR($CDCl_3$): ($CCl_3F$ standard)-191.0 (ddd, J = 22.5, 30.7, 54.3Hz)

$^1$HNMR ($CDCl_3$): δ1.9-2.4(m, 3H), 3.0-4.1(m, 4H), 4.2-5.1(m, 5H), 7.39(s, 10H)

IR (neat) 2160, 1500, 1460 cm $^{1}$

The spectrum data of the 4-fluoro derivative (11) are as follows:

Mass spectrum 326 (M $NH_2$):

$^1$HNMR ($CDCl_3$): δ1.7-1.9(m, 1H), 2.0-2.2(m, 1H), 2.3-2.5(m, 2H), 3.4-3.6(m, 2H), 3.8-3.9(m, 1H), 4.1-4.2-(m, 1H), 4.60(s, 4H), 7.2-7.5(m, 10H)

$^{19}$F-NMR($CDCl_3$.$CDCl_3F$ standard): -141.5(m)

300 mg of the 3-fluoro derivative (10) thus obtained was dissolved in ethanol (50 ml) and chloroform (2 ml), and 5% palladium-carbon (0.5 g) was added thereto. The mixture was hydrogenated to obtain an amino diol (12).

$^{19}$F-NMR($CDCl_3$): ($CCl_3F$ standard)-188.10 (ddd, J = 24.2, 33.4, 56.4Hz)

The crude product of the amino diol (12) was dissolved in 1-butanol (6 ml), and 300 mg (1.8 mmol) of 5-amino-4,6-dichloropyrimidine and triethylamine (0.5 ml) were added thereto. The mixture was refluxed for 18 hours under heating. The solvent was distilled off, and the residue was extracted with chloroform/water (1/1). The aqueous layer was passed through a column of Amberlite CG-100 (H type) (3 ml), and the desired product was eluted with 4% aqueous ammonia (200 ml). The solvent was distilled off to obtain 176 mg (yield: 81%, two steps) of a pyrimidine derivative.

176 mg (0.68 mmol) of the pyrimidine derivative was dissolved in ethyl orthoformate, and concentrated hydrochloric acid (0.5 ml) was added. The mixture was heated in an autoclave at 100°C for 18 hours. The solvent was distilled off, and 1N HCl (5 ml) was added thereto, and the mixture was stirred at room temperature for 3 hours. After the removal of the solvent, the mixture was purified by reversed phase C-18 silica gel chromatography to obtain 103 mg (yield: 61%) of 9-(3α-fluoro-2α-hydroxy-4β-hydroxymethyl-1β-cyclopentyl)-6-aminopurine (13).

Melting point: 196.2-199.2 (decomposed)

$^{19}$F-NMR(DMSO-$d_6$): ($CCl_3F$)-185.9 (ddd, J = 28.0, 30.0, 54.2Hz)

$^1$H NMR (DMSO-$d_6$): δ2.0-3.0(m, 3H), 3.0-3.7(m, 5H), 3.8-4.5(m, 2H), 5.45(d, J = 5.5Hz,1H), 7.19(brs, 2H), 8.11(s, 1H), 8.19(s, 1H)

On the other hand, 0.25 g (0.69 mmol) of the above-mentioned 4-fluoro derivative (11) was dissolved in ethanol (37 ml) and chloroform (1.6 ml), and 5% palladium-carbon (0.40 g) was added thereto. The mixture was hydrogenated to obtain 0.15 g of an amino diol (14).

The crude product of the amino diol (14) was dissolved in 1-butanol (6 ml), and 300 mg (1.8 mmol) of 5-amino-4,6-dichloropyrimidine and triethylamine (0.5 ml) were added thereto. The mixture was refluxed for 18 hours under heating. The solvent was distilled off, and the residue was extracted with chloroform,/water (1/1). The aqueous layer was passed through a column of Amberlite CG-120 (H type) (3 ml), and the desired compound was eluted with 4% aqueous ammonia (200 ml). The solvent was distilled off to obtain 0.15 g of a pyrimidine derivative.

0.15 g of the pyrimidine derivative was dissolved in ethyl orthoformate (6 ml), and concentrated

hydrochloric acid (0.5 ml) was added. The mixture was stirred at room temperature for 2 days. The solvent was distilled off, and a saturated methanol-ammonia (6 ml) was added to the residue at 0°C, and the mixture was heated in an autoclave at 100°C for 18 hours. The solvent was distilled off, and 1N HCl (5 ml) was added to the residue. The mixture was stirred at room temperature for 3 hours. The solvent was distilled off, and the residue was purified by reversed phase C-18 silica chromatography to obtain 80 mg of 9-(4α-fluoro-2α-hydroxy-4β-hydroxymethyl-1β-cyclopentyl)-6-aminopurine (15).

$^1$H-NMR ($CDCl_3$): δ2.0-3.0(m, 4H), 3.7-4.0(s, 1H), 4.8-5.0(m, 2H), 8.10(s, 1H), 8.25(s, 1H)

$^{19}$F-NMR ($D_2O.CCl_3F$ standard): -141.9(m)

EXAMPLE 2

Preparation of 1-(3α-fluoro-2α-hydroxy-4β-hydroxymethyl-1β-cyclopentyl)uracil (16) (a compound of the formula I wherein each of $R^1$ and $R^2$ is a hydrogen atom and B is a uracil residue)

180 mg (1.2 mmol) of 3α-fluoro-2α-hydroxy-4β-hydroxymethyl-1β-cyclopentylamine (12) (amine diol (12) obtained by the hydrogenation of the 3-fluoro derivative (10) obtained in Example 1) was dissolved in N,N-dimethylformamide (10 ml), and 3-ethoxy-2-propenoyl isocyanate (0.4M benzene solution, 3.0 ml, 1.2 mmol) was dropwise added over a period of 5 minutes. Ten minutes later, the mixture was returned to room temperature and then further heated at 30°C to distill off the solvent. By means of ethanol (5 ml × 3), low boiling substances were completely distilled off. Then, 10 ml of 2N hydrochloric acid was added to the residue, and the mixture was refluxed for 20 minutes under heating. After cooling to 0°C, the mixture was neutralized with 2N sodium hydroxide and then heated to 40°C to distill off water. The residue was purified by silica column chromatography to obtain 270 mg (yield: 92%) of the above identified compound (16).

$^{19}$F-NMR (acetone-$d_6$, $CCl_3F$ standard): -187.0 (ddd, J=24.2, 29.6, 53.7Hz)

$^1$H-NMR (acetone-$d_6$): δ1.8-2.6(m, 3H), 3.5-5.3(m, 5H), 5.70(d, J=7.9Hz, 1H), 8.00(d, J=7.9Hz, 1H)

EXAMPLE 3

Preparation of 1-(3α-fluoro-2α-hydroxy-4β-hydroxymethyl-1β-cyclopentyl)thymine (17) (a compound of the formula I wherein each of $R^1$ and $R^2$ is a hydrogen atom, and B is a thymine residue)

240 mg (1.6 mmol) of 3α-fluoro-α-hydroxy-4β-hydroxymethyl-1β-cyclopentylamine (12) was dissolved in N,N-dimethylformamide (10 ml), and 3-methoxy-2-methyl-2-propenoyl isocyanate (0.4M benzene solution, 4.0 ml, 1.6 mmol) was dropwise added over a period of 5 minutes. Ten minutes later, the mixture was returned to room temperature and then further heated to 30°C to distill off the solvent. By means of ethanol (5 ml × 3), low boiling substances were completely distilled off. Then, 10 ml of 2N hydrochloric acid was added thereto, and the mixture was refluxed for 20 minutes under heating. After cooling to 0°C, the mixture was neutralized with 2N sodium hydroxide and then heated to 40°C to distill off water. The residue was purified by silica gel column chromatography to obtain 460 mg (yield: 100%) of the above identified compound (17).

$^{19}$F-NMR (acetone-$d_6$, $CCl_3F$ standard): -186.9 (ddd, J=23.9, 29.8, 53.7Hz)

$^1$H-NMR (acetone-$d_6$): δ1.4-2.8(m+s (δ1.90), totally 5H), 3.5-5.2(m, 3H), 7.70(br s, 1H)

EXAMPLE 4

Preparation of 1-(3α-fluoro-2α-hydroxy-4β-hydroxymethyl-1β-cyclopentyl)cytosine (18) (a compound of the formula I wherein each of $R^1$ and $R^2$ is a hydrogen atom, and B is a cytosine residue)

98 mg (0.4 mmol) of the uracil derivative (16) prepared in Example 2 was dissolved in dry pyridine (10 ml), and 4-dimethylaminopyridine (10 ml) and dry acetic acid (2 ml) were added thereto. The mixture was stirred at room temperature for 1 hour. The reaction solution was poured into a 0.5 M potassium dihydrogen phosphate aqueous solution (20 ml) and extracted with chloroform (20 ml). The chlforoform layer was dried over anhydrous magnesium sulfate and then concentarted under reduced pressure. The residue was dissolved in dry acetonitrile (5 ml), and 270 mg (1.2 mmol) of 2-mesitylene sulfonyl chloride and 170 µl (1.2 mmol) of trimethylamine were added thereto. The mixture was stirred at room temperature

for 1 hour. The reaction solution was concentrated under reduced pressure, and a saturated ammonia-methanol solution (3 ml) was added to the residue. The mixture was stirred at room temperature for 15 hours. The reaction solution was concentrated under reduced pressure, then dissolved in water (5 ml), adsorbed on Amberlite CG-120 (H type), washed with water and then eluted with 5% aqueous ammonia to obtain the above identified compound (18). Yield: 66 mg (67%).

$^{19}$F-NMR ($D_2O$, $CCl_3F$ standard): -188.4 ppm (ddd, J = 22.46Hz, 32.23Hz, 54.69Hz)

$^{1}$H-NMR ($D_2O$): δ1.48-1.80(m, 1H), 2.32-2.82(m, 2H), 3.86(m, 2H), 4.80-5.44(m, 3H), 6.30(d, J = 7.5Hz, 1H), 7.94(d, J = 7.5Hz, 1H)

EXAMPLE 5

Preparation of 9-(3α-fluoro-2α-hydroxy-4β-hydroxymethyl-1β-cyclopentyl)guanine (19) (a compound of the formula I wherein each of $R^1$ and $R^2$ is a hydrogen atom, and B is a guanine residue)

236 mg (1.6 mmol) of 3α-fluoro-2α-hydroxy-4β-hydroxymethyl-1β-cyclopentylamine (12) was dissolved in 1-butanol (33 ml), and 518 mg (3.2 mmol) of 2-amino-4,6-dichloropyrimidine and 440 µl (3.2 mmol) of triethylamine were added thereto. The mixture was refluxed for 15 hours under heating. The reaction solution was cooled with ice, and the formed precipitates were removed by filtration. The filtrate was concentrated under reduced pressure, and the residue was dissolved in acetic acid (8 ml) and water (8 ml). Then, sodium acetate trihydrate (3.16 g) and 3.6 ml of an aqueous 4-chlorobenzene diazonium chloride solution (18 mmol)) were added thereto, and the mixture was stirred at room temperature for 15 hours. The formed precipitates were collected by filtration and dried. The precipitates were dissolved in a 50% ethanol aqueous solution (25 ml), and 1.3 ml of acetic acid and 1.3 g of zinc powder were added thereto. The mixture was stirred at 70°C for 3 hours. The reaction solution was filtered, and the filtrate was concentrated, then dissolved in water (20 ml) and washed with ethyl ether. The aqueous layer was adsorbed by Amberlite CG-120 (H type), then washed with water and eluted with 5% aqueous ammonia. The eluted solution was concentrated under reduced pressure to obtain a pyrimidine derivative (20). Yield: 106 mg (24%).

250 mg (0.85 mmol) of the pyrimidine derivative (20) was dissolved in N,N-dimethylformamide (2 ml), and triethyl orthoformate (15 ml) and concentrated hydrochloric acid (0.5 ml) were added thereto. The mixture was stirred at room temperature for 15 hours. The reaction solution was concentrated under reduced pressure, and 2N hydrochloric acid (20 ml) was added to the residue. The mixture was refluxed for 3 hours under heating. The reaction solution was left to cool, then adsorbed by Amberlite CG-120 (H type), washed with water and eluted with 5% aqueous ammonia and concentrated under reduced pressure to obtain the above identified compound (19). Yield: 90 mg (37%).

$^{19}$F-NMR ($CD_3OD$, $CCl_3F$ standard): -189.0 ppm(m)

$^{1}$H-NMR ($CD_3OD$): δ1.20-1.78(m, 1H), 1.98-2.01(m, 3H), 3.88(m, 2H), 4.72-5.50(m, 3H), 8.08(s, 1H)

EXAMPLE 6

Preparation of 9-(3α-fluoro-2α-hydroxyl-4β-hydroxymethyl-1β-cyclopentyl)-2,6-diaminopurine (21) (a compound of the formula I wherein each of $R^1$ and $R^2$ is a hydrogen atom, and B is a 2,6-diaminopurine residue)

250 mg (0.86 mmol) of the pyrimidine derivative (20) as the intermediate compound produced in Example 5, was dissolved in N,N-dimethylformamide (2 ml), and triethyl orthoformate (15 ml) and concentrated hydrochloric acid (0.5 ml) were added. The mixture was stirred at room temperature for 15 hours. The reaction solution was concentrated under reduced pressure, and a saturated ammonia-methanol solution (3 ml) was added to the residue. The mixture was left to stand at 70°C for 15 hours and concentrated under reduced pressure. The residue was dissolved in water (10 ml), then adsorbed by Amberlite (CG-120) (H type), washed with water and eluted with 5% aqueous ammonia. The eluted solution was concentrated under reduced pressure to obtain the above identified compound (21). Yield: 63 mg (30%).

$^{19}$F-NMR ($CD_3OD$): -173.77 ppm(m)

EXAMPLE 7

Preparation of 5-amino-3,6-dihydro-3-(3α-fluoro-2α-hydroxy-4β-hydroxymethyl-1β-cyclopentyl)-7H-1,2,3-triazolo[4,5-d]pyrimidin-7-one (22) (a compound of the formula I wherein each of $R^1$ and $R^2$ is a hydrogen atom, and B is a 5-amino-3,6-dihydro-7H-1,2,3-triazolo[4,5-d]pyrimidin-7-one residue)

250 mg (0.86 mmol) of the pyrimidine derivative (20) prepared in Example 5 was dissolved in 1N hydrochloric acid (2.5 ml), and 55 mg of sodium nitrite was added. The mixture was stirred under cooling with ice for 1 hour. The reaction solution was passed through Diaion SA-21A (OH type), and then 5 ml of 2N hydrochloric acid was added thereto. The mixture was refluxed for 3 hours under heating. This reaction solution was adsorbed by Amberlite CG-120 (H type), washed with water and eluted with 5% aqueous ammonia. The eluted solution was concentrated under reduced pressure to obtain the above identified compound (22). Yield: 60 mg.

$^{19}F$-NMR ($CD_3OD$, $CCl_3F$ standard): -188.7 ppm (ddd, J=21.71Hz, 32.57Hz, 55.43Hz)

$^1H$-NMR ($CD_3OD$): δ2.14-2.95(m, 3H), 3.88(m, 2H), 4.32(m, 1H), 4.54-5.44(m, 2H)

EXAMPLE 8

Preparation of 1-(4α-fluoro-2α-hydroxy-4β-hydroxymethyl-1β-cyclopentyl)uracil (23) (a compound of the formula I wherein each of $R^1$ and $R^2$ is a hydrogen atom, and B is a 2,6-diaminopurine residue)

58 mg (0.39 mmol) of 4α-fluoro-2α-hydroxy-4β-hydroxymethyl-1β-cyclopentylamine (14) (the amine diol (14) obtained by the hydrogenation of the 4-fluoro derivative (11) prepared in Example 1) was dissolved in N,N-dimethylformamide (1.5 ml), and 3-ethoxy-2-propenoyl isocyanate (0.4M benzene solution, 0.98 ml, 0.39 mmol) was dropwise added thereto over a period of 5 minutes. Ten minutes later, the mixture was returned to room temperature and the further heated to 30°C to distill off the solvent. By means of ethanol (2 ml × 2), the solvent was completely distilled off, and 2 ml of 2N hydrochloric acid was added to the residue. The mixture was refluxed for 20 minutes under heating. After cooling to 0°C, the mixture was neutralized with 2N sodium hydroxide and heated to 40°C to distill off water. The residue was purified by silica gel chromatography to obtain 53 mg (yield: 56%) of the above identified compound (23).

$^{19}F$-NMR ($CD_3OD$): -141.2(m)

$^1H$-NMR ($CD_3OD$): δ1.5-3.0(m, 4H), 3.5-4.0(m, 2H), 4 3-5.4(m, 2H), 5.95(d, J=7.9Hz, 1H), 7.95(d, J=7.9Hz, 1H)

EXAMPLE 9

Preparation of 1-(4α-fluoro-2α-hydroxy-4β-hydroxymethyl-1β-cyclopentyl)thymine (24) (a compound of the formula I wherein each of $R^1$ and $R^2$ is a hydrogen atom, and B is a thymine residue)

34 mg (0.23 mmol) of 4α-fluoro-2α-hydroxy-4β-hydroxymethyl-1β-cyclopentylamine (14) was dissolved in N,N-dimethylformamide (1.0 ml), and 3-methoxy-2-methyl-2-propenoyl isocyanate (0.4M benzene solution, 0.57 ml, 0.23 mmol) was dropwise added thereto over a period of 5 minutes. Ten minutes later, the mixture was returned to room temperature and further heated to 30°C to distill off the solvent. By means of ethanol (2 ml × 2), low boiling substances were completely distilled off, and then 2 ml of 2N hydrochloric acid was added thereto. The mixture was refluxed for 20 minutes under heating. After cooling to 0°C, the mixture was neutralized with 2N sodium hydroxide and heated to 40°C to distill off water. The product was purified by silica gel column chromatography to obtain 28 mg (yield: 47%) of the above identified compound (24).

$^{19}F$-NMR (acetone-$d_6$, $CCl_3F$): -138.3(m)

$^1H$-NMR (acetone-$d_6$): δ1.8-2.8(m+s(δ1.90), totally 7H), 3.4-4.0(m, 2H), 4.3-5.1(m, 2H), 7.65(br s, 1H)

EXAMPLE 10

Preparation of 1-(4α-fluoro-2α-hydroxy-4β-hydroxymethyl-1β-cyclopentyl)cytosine (25) (a compound of the formula I in which each of $R^1$ and $R^2$ is a hydrogen atom, and B is a cytosine residue)

400 mg (1.6 mmol) of the uracil derivative (23) prepared in Example 8 was dissolved in dry pyridine (10 ml), and 4-dimethylamino pyridine (10 mg) and dry acetic acid (2 ml) were added thereto. The mixture was stirred at room temperature for 15 minutes. The reaction solution was poured into a 0.5 M aqueous potassium dihydrogen phosphate (60 ml) and extracted with ethyl acetate (60 ml). The ethyl acetate layer was dried over anhydrous magnesium sulfate and then concentrated under reduced pressure. The residue was dissolved in dry acetonitrile (10 ml), and 1.08 g (4.9 mmol) of 1-methylenesulfonyl chloride and 680 µl (4.9 mmol) of triethylamine were added thereto. The mixture was stirred at room temperature for 15 minutes. The reaction solution was concentrated under reduced pressure, and a saturated ammonia-methanol solution (5 ml) was added to the residue. The mixture was stirred at room temperature for 15 hours. The reaction solution was concentrated under reduced pressure, and the residue was dissolved in water (30 ml), then adsorbed by Amberlite CG-120 ($H^+$ type), washed with water and eluted with 5% aqueous ammonia. The eluted solution was concentrated under reduced pressure to obtain the above identified compound (25). Yield: 174 mg (45%).

$^{19}$F-NMR ($D_2O$, $CCl_3F$): -143.5 ppm(m)

$^1$H-NMR ($D_2O$): δ1.94-3.14(m, 6H), 4.75(m, 1H), 4.84(m, 1H), 6.32(d, J=7.7Hz, 1H), 7.96(d, J=7.7Hz, 1H)

EXAMPLE 11

Preparation of 4β-acetamido-3β-hydroxy-2α-fluoro-1β-cyclopentane methyl acetate (26) (a compound of the formula I wherei $R^1$ is an acetyl group, $R^2$ is a hydrogen atom, and B is an acetamido group)

7.0 g (32.0 mmol) of 4β-acetamido-2β,3β-epoxycyclopentane-1β-methyl acetate (a compound of the formula III wherein $R^7$ is an acetyl group, and $R^6$ is an acetamido group) was dissolved in dichloromethane (100 ml), and the solution was cooled to 0°C. Then, 70% hydrogenfluoride-pyridine (18 ml) was dropwise added thereto over a period of 5 minutes. Two hours later, the reaction mixture was added to a saturated potassium carbonate aqueous solution and extracted with dichloromethane. The extract was purified by column chromatography to obtain 5.87 g (yield: 77%) of the above identified α-fluoroalcohol (26).

$^1$H-NMR ($CDCl_3$): δ1.4-1.6(m, 1H), 2.00(s, 3H), 2.07(s, 3H), 2.1-2.4(m, 2H), 4 07(d, J=6.8Hz, 2H), 4.1-5.1-(m, 4H), 6.52(d, J=7.2Hz, 1H)

$^{19}$F NMR ($CDCl_3$): ($CFCl_3$ standard)-179.9 (ddd, J=50.3, 27.8, 11.7Hz)

EXAMPLE 12

Preparation of 9-(3α-fluoro-2β-hydroxy-4β-hydroxy-methyl-1β-cyclopentyl)-6-aminopurine (28)

5.87 g (26.3 mmol) of the α-fluoroalcohol (26) prepared in Example 11, was dissolved in methanol (142 ml), and 2N HCl (142 ml) was added thereto. The mixture was refluxed for 1 hour under heating. Methanol was distilled off, and the residue was neutralized by passing it through Diaion SA-21A OH type (300 ml). The solvent was distilled off to obtain 3.6 g (yield: 92%) of an amino diol.

$^{19}$F-NMR ($D_2O$): ($CCl_3F$ standard): -181.0 (ddd, J=15.2, 28.8, 51.8Hz)

0.73 g (4.9 mmol) of the amino diol (27) obtained above was dissolved in 1-butanol (60 ml), and 2.0 g (12.2 mmol) of 5-amino-4,6-dichloropyridine and 3.5 ml of triethylamine were added thereto. The mixture was refluxed for 2 days under heating. The solvent was distilled off, and the residue was extracted with chloroform/water (1/1). The aqueous layer was adsorbed by Amberlite CG-120 (H type) (10 ml) and eluted with 0.3% aqueous ammonia (about 300 ml) to obtain 0.97 g (yield: 97%) of a diaminopyridine.

$^{19}$F-NMR ($CD_3OD$): ($CCl_3F$ standard): -179.1 (ddd, J=16.1, 27.2, 41.5Hz)

256 mg (1.0 mmol) of the diaminopyridine obtained above was dissolved in 25 ml of ethyl orthoformate, and concentrated hydrochloric acid (0.6 ml) was added thereto. The mixture was stirred at room temperature for 18 hours. The solvent was distilled off to obtain 182 mg (yield: 68%) of a chloropurine derivative.

$^{19}$F-NMR (acetone-$d_6$) ($CCl_3F$ standard): -175.3 (ddd, J=12.3, 26.4, 50.8Hz)

$^1$H-NMR (acetone-$d_6$): δ2.0-2.23(m, 2H), 2.3-4.0(m, 6H) 4.5-5.5(m, 2H), 8.59(s 1H), 8.73(s, 1H)

To 125 mg (0.47 mmol) of the chloropurine derivative, an ammonia-methanol solution (10 ml) saturated

at 0°C was added, and the mixture was heated in an autoclave at 100°C for 18 hours, and then cooled. Low boiling substances were distilled off. 1N HCl (5 ml) was added to the residue, and the mixture was stirred at room temperature for 3 hours. Then, the solvent was distilled off, and the residue was purified by reversed phase C-18 silica gel column chromatography to obtain 73 mg (yield: 63%) of the above identified fluoroadenosine (28).

Melting point: 200-210°C (decomposed)

$^{19}$F-NMR(DMSO-$d_6$): ($CCl_3F$ standard) -171.8 (ddd, J=12.6, 22.2, 50.0Hz)

$^{1}$H-NMR (DMSO-$d_6$): δ2.0-3.0(m, 3H), 3.0-5.2(m, 6H), 5.4-5.6(m, 1H), 7.16(brs, 2H), 8.13(brs, 2H)

EXAMPLE 13

Preparation of 1-(3α-fluoro-2β-hydroxy-4β-hydroxy methyl-1β-cyclopentyl)uracil (29)

300 mg (2.0 mmol) of the amino diol (27) in Example 12 was dissolved in 10 ml of N,N-dimethylformamide, and the solution was cooled to -25°C. Then, 3-ethoxy-2-propenoyl isocyanate (0.4M benzene solution, 5.0 ml, 2.0 mmol) was dropwise added thereto over a period of 5 minutes. Ten minutes later, the mixture was returned to room temperature, and the solvent was distilled off over a warm bath at 30°C. By means of ethanol (5 ml × 3) the solvent was completely distilled off. Then, 10 ml of 2N hydrochloric acid was added, and the mixture was refluxed for 20 minutes under heating. After cooling to 0°C, the mixture was neutralized by 2N sodium hydroxide, and water was distilled off at a bath temperature of 40°C. The residue was purified by silica gel column chromatography to obtain 340 mg (yield: 70%) of the above identified compound (29).

$^{19}$F-NMR ($CD_3OD$, $CCl_3F$ standard): -175.3 (ddd, J=12.7, 28.3, 51.0Hz)

$^{1}$H-NMR ($CD_3OD$): δ1.9-2.8(m, 3H), 3.6-4.0(m, 2H), 4.3-5.4(m, 3H), 5.85(d, J=8.6Hz, 1H), 7.98(d, J=8.6Hz, 1H)

EXAMPLE 14

Preparation of 1-(3α-fluoro-2β-hydroxyl-4β-hydroxylmethyl-1β-cyclopentyl)thymine (30)

270 mg (1.8 mmol) of the amino diol (27) prepared in Example 12 was dissolved in 10 ml of N,N-dimethylformamide. The solution was cooled to -25°C. Then, 3-methoxy-2-methyl-2-propanoyl isocyanate (0.4M benzene solution, 4.5 ml, 1.8 mmol) was dropwise added thereto over a period of 5 minutes. Ten minutes later, the mixture was returned to room temperature and the solvent was distilled off by adjusting the warm bath at 30°C. By means of ethanol (5 ml × 3), the solvent was completely distilled off, and then 10 ml of 2N hydrochloric acid was added to the residue. The mixture was refluxed for 20 minutes under heating. After cooling to 0°C, the mixture was neutralized with 2N sodium hydroxide, and water was distilled off under a warm bath temperature of 40°C. The product was purified by silica gel colum chromatography to obtain 380 mg (yield: 82%) of the above identified compound (30).

$^{19}$F-NMR ($CD_3OD$, $CCl_3F$ standard): -175.0 (ddd, J=12.7, 28.3, 51.0Hz)

$^{1}$H-NMR ($CD_3OD$): δ1.8-2.8(m+s , δ2.02(s, 3H), totally 6H), 3.7-4.0(m, 3H), 4.2-4.6(m, 2H), 7.80(br s, 1H)

EXAMPLE 15

Preparation of 1-(3α-fluoro-2β-hydroxy-4β-hydroxylmethyl-1β-cyclopentyl)-5-iodouracil (32)

820 mg (3.4 mmol) of the uracil derivative (29) prepared in Example 13, was dissolved in dry pyridine (10 ml), and 4-dimethylaminopyridine (5 mg) and dry acetic acid (1 ml) were added thereto. The mixture was stirred at room temperature for 30 minutes. The reaction solution was poured into a 0.5M potassium dihydrogenphosphate aqueous solution (60 ml) and extracted with chloroform (60 ml). The chloroform layer was dried over anhydrous magnesium sulfate and then concentrated under reduced pressure. The residue was purified by silica gel chromatography to obtain an acetyl derivative (31). Yield: 1.13 g (100%).

560 mg (1.7 mmol) of this acetyl derivative (31) was dissolved in acetic acid (10 ml), and 220 mg (0.87 mmol) of iodine was added. Then, fuming nitric acid was added until the color of iodine disappeared. This reaction solution was concentrated under reduced pressure, and the residue was dissolved in chloroform

(50 ml) and washed twice with a saturated sodium carbonate aqueous solution (50 ml). The chloroform layer was concentrated under reduced pressure and then dissolved in ethanol (30 ml). Then, 2N sodium hydroxide (30 ml) was added thereto, and the mixture was stirred under cooling with ice for 20 minutes. The reaction solution was neutralized with 2N hydrochloric acid and then concentrated under reduced pressure. The product was purified by silica gel column chromatography to obtain the above identified compound (32). Yield: 35%.

$^{19}$F-NMR ($CD_3OD$, $CCl_3F$ standard): -192.7 ppm (dt, J = 20.00Hz, 54.86Hz)

$^{1}$H-NMR ($CD_3OD$): δ1.24-1.96(m, 3H), 3.92(bd, J = 4.63Hz, 2H), 4.43(m, 1H), 4.50-5.52(m, 2H), 7.95(d, J = 1.80Hz, 1H)

EXAMPLE 16

Preparation of 1-(3α-fluoro-2β-hydroxyl-4β-hydroxymethyl-1β-cyclopentyl)cytosine (33)

560 mg (1.7 mmol) of the acetyl derivative (31) formed during the preparation in Example 15 was dissolved in dry acetonitrile (10 ml), and 1.08 g (5.1 mmol) of 2-mesitylene sulfonyl chloride was added thereto. The mixture was stirred at room temperature for 30 minutes. The reaction solution was concentrated under reduced pressure. Then, a saturated ammonia-methanol solution (10 ml) was added thereto, and the mixture was stirred at room temperature for 15 hours. This reaction solution was concentrated under reduced pressure and then dissolved in a 50% methanol aqueous solution (10 ml). The solution was adsorbed by Amberlite CG-120 (H type), then washed with water and eluted with 5% aqueous ammonia. The eluted solution was concentrated under reduced pressure to obtain the above identified compound (33). Yield: 310 mg (77%).

$^{19}$F-NMR ($CD_3OD$, $CCl_3F$ standard): -174.3 ppm (ddd, J = 12.00Hz, 29.71Hz, 52.57Hz)

$^{1}$H-NMR ($CD_3OD$): δ1.96-2.68(m, 3H), 3.88(bd, J = 4.97Hz, 2H), 4.28-4.64(m, 1H),5.00-5.42(m, 2H), 6.08(d, J = 5.14Hz, 1H), 7.98(d, J = 5.14Hz, 1H)

EXAMPLE 17

Preparation of (3α-fluoro-2β-hydroxy-4β-hydroxymethyl)-(2,5-diamino-6-chloro-4-pyrimidinyl)amine (34)

412 mg (2.76 mmol) of 3α-fluoro-2β-hydroxy-4β-hydroxymethyl-1β-cyclopentylamine (27) was dissolved in 1-butanol (58 ml), and 906 mg (5.52 mmol) of 2-amine-4,6-dichloropyrimidine and 770 µl (5.52 mmol) of triethylamine were added thereto. The mixture was refluxed for 15 hours under heating. The reaction solution was cooled with ice. Formed precipitates were removed by filtration, and the filtrate was concentrated under reduced pressure. The residue was suspended in acetic acid (14 ml) and water (14 ml), and sodium acetate trihydrate (5.52 g) and 6.4 ml (3.2 mmol of 4-chlorobenzene diazoniumchloride were added thereto. The mixture was stirred at room temperature for 15 hours. Formed precipitates were collected by filtration and dried. To the precipitates, ethanol (2.2 ml) and zinc powder (2.2 g) were added, and the mixture was stirred at 70°C for 1 hour. The reaction solution was filtered, and the filtrate was concentrated under reduced pressure, then dissolved in water (50 ml) and washed with ethyl ether. This aqueous layer was adsorbed by Amberlite CG-120 (H type), then washed with water and eluted with 5% aqueous ammonia. The eluted solution was concentrated under reduced pressure to obtain the above identified comound (34). Yield: 120 mg.

IR (film) 3300 cm $^{1}$

EXAMPLE 18

Preparation of 9-(3α-fluoro-2β-hydroxy-4β-hydroxymethyl-1β-cyclopentyl)guanine (35)

300 mg (1.03 mmol) of the pyrimidine derivative (34) prepared in Example 17 was dissolved in N,N-dimethylformamide (2 ml), and triethyl orthoformate (15 ml) and concentrated hydrochloric acid (0.5 ml) were added thereto. The mixture was stirred at room temperature for 15 hours. The reaction solution was concentrated, and 2N hydrochloric acid (20 ml) was added to the residue. The mixture was refluxed for 3 hours under heating. This reaction solution was adsorbed by Amberlite CG-120 (H type), washed with

water and eluted with 5% aqueous ammonia. The eluted solution was concentrated under reduced pressure to obtain the above identified compound (35).

Yield: 126 mg (43%).

$^{19}$F-NMR ($CD_3OD$, $CCl_3F$ standard): -174.2 ppm (ddd, J=11.43Hz, 32.57Hz, 52.00Hz)

$^1$H-NMR ($CD_3OD$): δ1.22-1.83(m, 1H), 2.05-2.95(m, 3H), 3.88(m, 2H), 4.35-5.25(m, 3H), 9.11(s, 1H),

## EXAMPLE 19

Preparation of 9-(3α-fluoro-2β-hydroxyl-4β-hydroxymethyl-1β-cyclopentyl)-2,6-diaminopurine (36)

300 mg (1.03 mmol) of the pyrimidine derivative (34) prepared in Example 17 was dissolved in N,N-dimethylformamide (2 ml), and triethyl orthoformate (15 ml) and concentrated hydrochloric acid (0.5 ml) were added thereto. The mixture was stirred at room temperature for 15 hours. This reaction solution was concentrated under reduced pressure, and a saturated ammonia-methanol solution (10 ml) was added to the residue. The mixture was left to stand at 70°C for 15 hours. The reaction solution was concentrated under reduced pressure, and the residue was dissolved in water (5 ml), adsorbed by Amberlite CG-120 (H type), washed with water and eluted with 5% aqueous ammonia. The eluted solution was concentrated under reduced pressure to obtain the above identified compound (36).

Yield: 138 mg (47%).

$^{19}$F-NMR ($CD_3OD$, $CCl_3F$ standard): -189.13 ppm(m)

## EXAMPLE 20

Preparation of 5-amino-3,6-dihydro-3-(3α-fluoro-2β-hydroxy-4β-hydroxymethyl-1β-cyclopentyl)-7H-1,2,3-triazolo[4,5-d]pyrimidin-7-one (37)

300 mg (1.03 mmol) of the pyrimidine derivative (34) prepared in Example 17 was dissolved in 1N hydrochloric acid (2.5 ml), and sodium nitrite (65 mg) was added thereto. The mixture was stirred under cooling with ice for 1 hour. The reaction solution was passed through Diaion SA-21A (OH type), and 2N hydrochloric acid was added thereto. The mixture was refluxed for 3 hours under heating. This reaction solution was adsorbed by Amberlite CG-120 (H type), washed with water and eluted with 5% aqueous ammonia. The eluted solution was concentrated under reduced pressure to obtain the above identified compound (37). Yield: 121 mg.

$^{19}$F-NMR ($CD_3OD$, $CCl_3F$ standard): -178.8 ppm (ddd, J=14.29Hz, 32.00Hz, 54.57Hz)

$^1$H-NMR ($CD_3OD$): δ2.12-2.89(m, 3H), 3.82(d, J=6.17Hz, 2H), 4.10-4.38(m, 1H), 4.62-5.54(m, 2H)

## EXAMPLE 21

Preparation of 9-(3α-fluoro-2α-hydroxyl-4β-hydroxymethyl-1β-cyclopentyl)-6-aminopurine (38)

200 mg (0.75 mmol) of aristeromycine i.e. 9-(2α,3α-hydroxy-4β-hydroxymethyl-1β-cyclopentyl)-6-aminopurine was dissolved in acetonitrile (10 ml), and 1.4 ml of 2-acetoxy isobutyryl bromide was added thereto. The mixture was reacted under reflux for 1 hour. Acetonitrile was distilled off, and a saturated sodium hydrogen carbonate aqueous solution was added to the residue. The mixture was extracted with ethyl acetate. The crude product was dissolved in methanol (5 ml). A 1M methanol solution of sodium methoxide (2.0 ml) was added thereto, and the mixture was reacted at room temperature for 1.5 hours. The solvent was distilled off, and water (10 ml) was added to the residue. The mixture was neutralized with 2N acetic acid. The crude product was separated by C-18 reversed phase column chromatography to obtain 0.17 g of an epoxy derivative of the formula III wherein $R^7$ is a hydrogen atom, $R^6$ is an adenine residue, and the epoxy group takes an α-configuration.

$^1$H-NMR ($CDCl_3$): δ2.0-2.8(m, 3H), 3.7-3.9(m, 2H), 3.9-4.2(m, 2H), 5.2-5.4(m, 1H), 8.50(s, 1H), 8.60(s, 1H)

The epoxy derivative thus prepared was dissolved in tetrahydrofuran (3 ml), and the solution was added to a suspension of 30 mg (0.6 mmol) of sodium hydride (60%) in tetrahydrofuran (3 ml), and the mixture was stirred at room temperature for 10 minutes. Then, 0.07 ml (0.6 mmol) of benzyl bromide was added thereto, and the mixture was refluxed for 4 hours under heating. The reaction solution was cooled, and after

an addition of water, extracted with dichloromethane. The crude product was dissolved in dichloromethane (10 ml). Then, 0.5 ml of a hydrogen fluoride-pyridine complex was added thereto, and the mixture was stirred at 0°C for 45 minutes. The reaction mixture was poured into a saturated potassium carbonate solution, and extracted with dichloromethane. The crude product was dissolved in ethanol (5 ml) and chloroform (0.25 ml), and 50 mg of palladium-carbon (5%) was added thereto. The mixture was stirred at room temperature for 18 hours, and then subjected to column chromatography to obtain 35 mg of the above identified compound (38).

$^{19}$F-NMR ($CDCl_3$, $CCl_3F$ standard): -186.3 (m)

EXAMPLE 22

(1) Preparation of 9-(3α-fluoro-2α-hydroxy-4β-t-butyldimethylsiloxymethyl-1β-cyclopentyl)-6-dimethylaminomethylene aminopurine (39)

The above identified compound (39) was prepared by using 9-(3α-fluoro-2α-hydroxy-4β-hydroxymethyl-1β-cyclopentyl)-6-aminopurine (13) prepared in Example 1.

670 mg (2.7 mmol) of fluoroadenosine (13) was dissolved in dimethylformamide (12 ml), and 2.03 ml (15.3 mmol) of dimethylformamide dimethylacetal was added thereto. The mixture was stirred at room temperature for 2 hours. The reaction solution was concentrated under reduced pressure, and the residue was purified by silica gel column chromatography. Yield: 680 mg (2.24 mmol, 82.9%).

520 mg (1.71 mmol) of the product was dissolved in dimethylformamide (3 ml), and 233 mg (3.42 mmol) of imidazole was added thereto and cooled with ice. A dimethylformamide solution of t-butyldimethylsilyl chloride (258 mg/2 ml) was dropwise added thereto, and the mixture was stirred at room temperature for 10 hours.

The reaction solution was dissolved in 50 ml of benzene and washed with water. The benzene layer was dried and concentrated under reduced pressure. The residue was purified by silica gel column chromatography to obtain a protected fluoroadenosine (39). Yield: 264 mg (0.63 mmol, 37%), recovery of the starting material: 250 mg (0.82 mmol, 47.9%).

$^{1}$H-NMR ($CDCl_3$): δ9 07(s, 1H), 8.67(s, 1H), 7.96(s, 3H), 5.31-3.40(m, 7H), 3.24(s, 3H), 3.19(s, 3H), 0.88(s, 9H), 0(s, 6H)

$^{19}$F-NMR ($CDCl_3$, $CCl_3F$ standard): -191.3 ppm (ddd, J = 25.7, 32.6, 56.5Hz)

(2) Preparation of 9-(3α-fluoro-4β-hydroxymethyl-1β-cyclopentyl)-6-aminopurine (40)

230 mg (0.52 mmol) of the alcohol (39) prepared in Step (1) was dissolved in dichloromethane (5 ml), and 190 mg (1.56 mmol) of 4-dimethylaminopyridine was added thereto. The mixture was cooled to 0°C, and 170 mg (1.0 mmol) of phenylchlorothionocarbonate was added thereto. The mixture was stirred at room temperature for 15 minutes. The mixture was treated with water in a usual manner, and then subjected to silica gel column chromatography to obtain a crude product. The crude product was dissolved in benzene (10 ml), and tributyltin hydride (1.0 ml) and N,N'-azobisisobutyronitrile (5 mg) was added to the solution. The mixture was reacted under reflux for 15 minutes. The reaction mixture was concentrated, and purified by silica gel chromatography to obtain a crude product.

The silyl ether thus obtained was dissolved in tetrahydrofuran (10 ml), and tetrabutylammonium fluoride (1M solution, 1.0 ml) was added. The mixture was stirred at room temperature for 1 hour. The desilyl derivative was purified by column chromatography. The product was dissolved in pyridine/28% aqueous ammonia/water (1/1/4.5 ml), and the solution was stirred at room temperature for 18 hours. The reaction mixture was concentrated and purified by column chromatography (chloroform/methanol = 4/1) to obtain 90 mg of the above identified compound (40).

$^{1}$H-NMR ($D_2O$): δ1.6-2.0(m, 5H), 2.75(d, J = 5.4Hz, 2H), 3.8-4.6(m, 2H), 7.19(s, 1H), 7.24(s, 1H)

$^{19}$F-NMR ($D_2O$, $CDCl_3$, $CCl_3F$ standard): -165.9 (dddd, J = 52.1, 34.5, 30.5, 18.7Hz)

EXAMPLE 23

Preparation of 4β-acetamido-2α-fluoro-1β-cyclopentane methyl acetate (41) (Step (1))

To 219 mg of N-bromosuccinic acid imide, 1 ml of dry ethyl ether was added. The mixture was cooled to 0°C, and 1 ml of a HF-pyridine solution (70%) was added thereto. Then, a solution of 243 mg of cis-4β-acetamidocyclopent-2-ene methyl acetate in dry ethyl ether (1 ml) was added thereto at 0°C, and the mixture was stirred at 0°C for 30 minutes and at room temperature for 2 hours. The reaction solution was diluted with chloroform, then poured into a saturated sodium hydrogencarbonate solution and extracted with chloroform. The chloroform layer was dried, concentrated and subjected to purification by silica gel chromatography (chloroform/methanol = 50/1) to obtain 280 mg of the above identified compound.

$^{19}$F-NMR($CDCl_3$): -154.0 (ddd, J = 49.6, 33.9, 14.7Hz)

$^{1}$H-NMR ($CDCl_3$): δ1.3-2.8(m, 3H), 2.03(s, 3H), 2.09(s, 3H), 4.2-5.5(m, 5H), 5.9(br, s, 1H)

IR($CDCl_3$): 3040, 1750, 1690, 1520 cm $^{1}$

To a solution of 278 mg of the bromide thus obtained, in dry benzene (20 ml), 5 mg of α,α'-azobisisobutyronitrile and 1.25 ml of tributyltin hydride were added. The mixture was refluxed at 80°C for 30 minutes under heating. The solvent was distilled off, and the residue was purified by silica gel column chromatography (chloroform/methanol = 50/1) to obtain 180 mg of the above identified compound (41).

$^{18}$F-NMR($CDCl_3$): -171.1 (dddd, J = 52.7, 33.2, 28.6, 21.7Hz)

$^{1}$H-NMR ($CDCl_3$): δ1.00-2.60(m, 5H), 1.96(s, 3H), 2.08(s, 3H), 4.00-5.40(m, 4H), 5.90(br, s, 1H)

IR($CDCl_3$): 1743, 1680, 1520 cm $^{1}$

EXAMPLE 24

Preparation of 4β-acetamido-2α-fluoro-1β-cyclopentane methyl acetate (41) (Step (2))

To 430 mg of 4β-acetamido-3β-hydroxy-2α-fluoro-1β-cyclopentane methyl acetate (26) prepared in Example 11, 37 ml of toluene, 1.45 g of triphenylphosphine, 936 mg of iodine and 360 mg of imidazole were added, and the mixture was refluxed for 4 hours under heating. The reaction mixture was left to cool, poured into a saturated sodium hydrogencarbonate aqueous solution and extracted with chloroform. The extract was concentrated, dried and subjected to purification by column chromatography to obtain 55 mg of iodide.

$^{19}$F-NMR($CDCl_3$): -166.0 (ddd, J = 51.8, 27.3, 26.6Hz)

$^{1}$H-NMR ($CDCl_3$): δ1.20-2.60(m, 3H), 1.93(s, 3H), 2.03(s, 3H), 4.00-5.50(m, 5H)

To a solution of 55 mg of the iodide thus obtained, in dry benzene (3 ml), 1 mg of α,α'-azodiisobutyronitrile and 0.23 ml of tributyltin hydride were added, and the mixture was refluxed at 80°C for 20 minutes. The solvent was distilled off and the residue was purified by silica gel column chromatography to obtain 37 mg of the above identified compound (41).

EXAMPLE 25

Preparation of 4β-amino-2α-fluoro-1β-cyclopentane methanol (42)

To 169 mg of the fluoride (41) prepared in Example 23, 4 ml of 2N hydrochloric acid was added, and the mixture was stirred at 100°C for 8 hours. The product was purified by Diaion SA-11a to obtain 92 mg of the above identified compound (42).

The fluoride (41) obtained in Example 24 was subjected to the same treatment as above, whereby the same compound (42) as identified above was obtained.

$^{19}$F-NMR ($D_2O$): -165.1 (dddd, J = 54.0, 36.6, 31.3, 22.7Hz)

$^{1}$H-NMR ($D_2O$): δ0.80-2.40(m, 5H), 3.40-5.20(m, 4H)

EXAMPLE 26

Preparation of 1-(3α-fluoro-4β-hydroxymethyl-1β-cyclopentyl)uracil (43)

130 mg (0.98 mmol) of 3α-fluoro-4β-hydroxymethyl-1β-cyclopentylamine (42) prepared in Example 25 was dissolved in N,N-dimethylformamide (5 ml), and 3-ethoxy-2-propenoyl isocyanate (0.4M benzene solution, 2.5 ml, 1.0 mmol) was dropwise added thereto over a period of 5 minutes. Ten minutes later, the mixture was returned to room temperature and further heated to 30°C to distill off the solvent. By means of ethanol (5 ml × 2), low boiling substances were completely distilled off, and then 10 ml of 2N hydrochloric acid was added to the residue. The mixture was refluxed for 20 minutes under heating. After cooling to 0°C, the mixture was neutralized with 2N sodium hydroxide and heated to 40°C to distill off water. The product was purified by silica gel column chromatography to obtain 150 mg (yield: 67%) of the above identified compound (43).

$^{19}$F-NMR ($D_2O$, $CCl_3F$): -159.2(m)

$^{1}$H-NMR ($D_2O$): δ1.6-3.0(m, 5H), 3.8-4.0(m, 2H), 4.9-5.8(m, 1H), 6.10(d, J=7.9Hz, 1H), 8.00(d, J=7.9Hz, 1H)

EXAMPLE 27

Preparation of 1-(3α-fluoro-4β-hydroxymethyl-1β-cyclopentyl)thymine (44)

200 mg (1.5 mmol) of 3α-fluoro-4β-hydroxymethyl-1β-cyclopentylamine (42) was dissolved in N,N-dimethylformamide (7 ml), and 3-methoxy-2-methyl-2-propenoyl isocyanate (0.4M benzene solution, 3.8 ml, 1.5 mmol) was dropwise added thereto over a period of 5 minutes. Ten minutes later, the mixture was returned to room temperature and further heated to 30°C to distill off the solvent. By means of ethanol (4 ml × 3), low boiling substances were completely distilled off. Then, 10 ml of 2N hydrochloric acid was added to the residue, and the mixture was refluxed for 20 minutes under heating. After cooling to 0°C, the mixture was neutralized with 2N sodium hydroxide and heated to 40°C to distill off water. The product was purified by silica gel column chromatography to obtain 350 mg (yield: 96%) of the above identified compound (44).

$^{19}$F-NMR (acetone-$d_6$, $CCl_3F$ standard): -165.2(m)

$^{1}$H-NMR (acetone-$d_6$): δ1.6-2.7(m+s(δ1.88), totally 8H), 3.0-5.6(m, 4H), 7.68(br s, 1H)

EXAMPLE 28

Preparation of 1-(3α-fluoro-4β-hydroxymethyl-1β-cyclopentyl)cytosine (45)

240 mg (1.0 mmol) of the uracil derivative (43) prepared in Example 26 was dissolved in dry pyridine (10 ml), and 4-dimethylaminopyridine (10 mg) and dry acetic acid (1 ml) were added thereto. The mixture was stirred at room temperature for 30 minutes. The reaction solution was poured into a 0.5M potassium dihydrogen phosphate aqueous solution (50 ml) and extracted with ethyl acetate (50 ml). The ethyl acetate layer was dried over anhydrous magnesium sulfate and concentrated under reduced pressure. The residue was dissolved in dry acetonitrile (10 ml), and 670 mg (3.0 mmol) of 2-mesitylene sulfonyl chloride and 420 µl (3.0 mmol) of triethylamine were added thereto. The mixture was stirred at room temperature for 50 minutes. The reaction solution was concentrated under reduced pressure, and the residue was dissolved in a saturated ammonia-methanol solution (3 ml) and stirred at room temperature for 15 hours. The reaction solution was concentrated under reduced pressure, and the residue was dissolved in a 50% methanol aqueous solution (20 ml), adsorbed by Amberlite CG-120 (H type), washed with water and eluted with 5% aqueous ammonia. The eluted solution was concentrated under reduced pressure to obtain the above identified compound (45). Yield: 55 mg (24%).

$^{19}$F-NMR ($CD_3OD$, $CCl_3F$ standard): -167.5 ppm (ddt, J=22.95Hz, 30.76Hz, 53.22Hz)

$^{1}$H-NMR ($CD_3OD$): δ1.64-2.78(m, 5H), 3.83(bd, 2H), 5.26(dm, J=53.22Hz, 1H), 5.38(m, 1H), 6.13(d, J=7.5Hz, 1H), 7.95(d, J=7.5Hz, 1H)

EXAMPLE 29

(1) Preparation of 4α-(2,5-diamino-6-chloro-4-pyrimidinylamino)-2β-fluoro-1α-cyclopentane methanol (46)

910 mg (6.8 mmol) of 3α-fluoro-4β-hydroxymethyl-1β-cyclopentylamine (42) was dissolved in 1-butanol (30 ml), and 2.49 g (15.22 mmol) of 2-amino-4,6-dichloropyrimidine and 2.1 ml (15.2 mmol) of triethylamine were added thereto. The mixture was refluxed for 15 hours under heating. The reaction mixture was cooled with ice, and formed precipitates were removed by filtration. The filtrate was concentrated under reduced pressure. The residue was dissolved in acetic acid (34 ml) and water (34 ml) and 13.6 g of sodium acetate trihydrate and 16 ml (7.5 mmol) of 4-chlorobenzene diazoniumchloride solution were added thereto. The mixture was stirred at room temperature for 15 hours. Formed precipitates were, collected by filtration and dried. To the precipitates, a 50% ethanol aqueous solution (100 ml) and acetic acid (11 ml) were added, and then 11 g of zinc powder was added. The mixture was stirred at 70°C for 1 hour. The reaction solution was filtered, then adsorbed by Amberlite CG-120 (H type), washed with water and eluted with 5% aqueous ammonia. The eluted solution was concentrated under reduced pressure to obtain the above identified compound (46). Yield: 380 mg (20%).

IR (film): 330 cm $^{1}$

(b) Preparation of 9-(3α-fluoro-4β-hydroxymethyl-1β-cyclopentyl)guanine (47)

190 mg (0.69 mmol) of the pyrimidine derivative (46) prepared in the above Step (a) was dissolved in N,N-dimethylformamide (2 ml), and triethyl orthoformate (30 ml) and concentrated hydrochloric acid (1 ml) were added thereto. The mixture was stirred at room temperature for 48 hours. The reaction solution was concentrated under reduced pressure, and 2N hydrochloric acid (20 ml) was added to the residue. The mixture was refluxed for 4 hours under heating. This reaction solution was adsorbed by Amberlite CG-120 (H type) and eluted with 5% aqueous ammonia. The eluted solution was concentrated under reduced pressure to obtain the above identified compound (47). Yield: 101 mg (55%).

$^{19}$F-NMR ($CD_3OD$, $CCl_3F$ standard): -167.5(m)

$^{1}$H-NMR ($CD_3OD$): δ1.91-2.89(m, 5H), 3.87(bd, 2H), 5.02-5.68(m, 2H), 8.09(s, H)

EXAMPLE 30

Preparation of 9-(3α-fluoro-4β-hydroxymethyl-1β-cyclopentyl)-2,6-diaminopurine (49)

190 mg (0.69 mmol) of the pyrimidine derivative (46) prepared in Step (a) in Example 29 was dissolved in N,N-dimethylformamide (2 ml), and triethyl orthoformate (30 ml) and concentrated hydrochloric acid (1 ml) were added thereto. The mixture was stirred at room temperature for 48 hours. The reaction solution was concentrated under reduced pressure, and a saturated ammonia-methanol solution (3 ml) was added to the residue. The mixture was left to stand at 70°C for 15 hours. The reaction solution was concentrated under reduced pressure, dissolved in water (10 ml), then adsorbed by Amberlite CG-120 (H type), washed with water and eluted with 5% aqueous ammonia. The eluted solution was concentrated under reduced pressure to obtain the above identified compound (49). Yield: 147 mg (40%).

$^{19}$F-NMR ($CD_3OD$, $CCl_3F$ standard): -167.3 ppm(m)

$^{1}$H-NMR ($CD_3OD$): δ1.89-2.86(m, 4H), 3.88(bd, 2H), 5.04-5.66(m, 2H), 8.14(s, 1H)

**Claims**

1. A 1β-substituted-2-hydroxy(or unsubstituted)-3(or 4)-fluoro-4β-hydroxymethyl cyclopentane derivative having the formula:

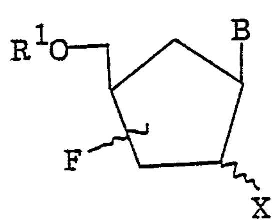

(I)

wherein B is a residue of a nucleic acid base, an azido group, an amino group or an amino group protected by a protecting group; $R^1$ is a hydrogen atom or a hydroxyl-protecting group; and X is a hydrogen atom or $-OR^2$ wherein $R^2$ is a hydrogen atom or a hydroxyl-protecting group, provided that the fluorine atom F is present at the 3α-, 3β-or 4α-position.

2. The derivative according to Claim 1, which is a 1β-substituted-2α-hydroxy-3α-fluoro-4β-hydroxymethyl cyclopentane derivative.

3. The derivative according to Claim 1, which is a 1β-substituted-2α-hydroxy-4α-fluoro-4β-hydroxymethyl cyclopentane derivative.

4. The derivative according to Claim 1, which is a 1β-substituted-2β-hydroxy-3α-fluoro-4β-hydroxymethyl cyclopentane derivative.

5. The derivative according to Claim 1, which is a 1β-substituted-2α-hydroxy-3β-fluoro-4β-hydroxymethyl cyclopentane derivative.

6. The derivative according to Claim 1, which is a 1β-substituted-3α-fluoro-4β-hydroxymethyl cyclopentane derivative.

7. The derivative according to Claim 1, which is a 1β-substituted-3β-fluoro-4β-hydroxymethyl cyclopentane derivative.

8. The derivative according to Claim 1, wherein B is a residue of a nucleic acid base; $R^1$ is a hydrogen atom; and X is a hydroxyl group.

9. The derivative according to Claim 1, wherein B is a residue of a nucleic acid base; $R^1$ is a hydrogen atom; and X is a hydrogen atom.

10. The derivative according to Claim 1, wherein the residue of a nucleic acid base is a substituted purine residue.

11. The derivative according to Claim 10, wherein the substituted purine residue is a residue of a compound selected from the group consisting of adenine and guanine which may be substituted.

12. The derivative according to Claim 1, wherein the residue of a nucleic acid base is a substituted pyrimidine residue.

13. The derivative according to Claim 12, wherein the substituted pyrimidine residue is a residue of a compound selected from the group consisting of uracil, cytosine and thymine which may be substituted.

14. A process for producing a 1β-substituted-2α-hydroxy-3α(or 4α)fluoro-4β-hydroxymethyl cyclopentane derivative of the formula:

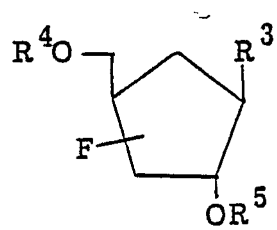

(I-1)

wherein $R^3$ is an azido group or an amino group protected by a protecting group; and each of $R^4$ and $R^5$ is a hydroxyl-protecting group, provided that the fluorine atom F is present at the 3α-or 4α-position, which comprises fluorinating a cyclopentane triol derivative of the formula:

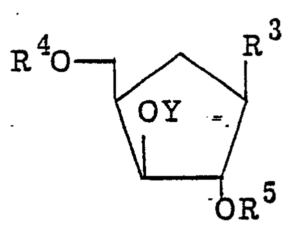

(II)

wherein $R^3$, $R^4$ and $R^5$ are as defined above, and Y is a hydrogen atom or an active group, with a fluorinating agent.

15. A process for producing a 1β-substituted-2α-hydroxy-3α(or 4α)fluoro-4β-hydroxymethyl cyclopentane derivative of the formula:

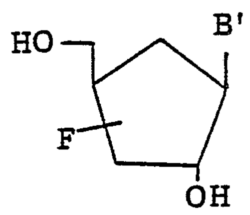

(I-2)

wherein B′ is a residue of a nucleic acid base, provided that the fluorine atom F is present at the 3α-or 4α-position, which comprises converting $R^3$ of a cyclopentane derivative of the formula I-1 obtained by the process of Claim 14 to an amino group, simultaneously or subsequently removing the protecting group, and then converting the amino group to a nucleic acid base.

16. A process for producing a 1β-substituted-2-hydroxy-3-fluoro-4β-hydroxymethyl cyclopentane derivative of the formula:

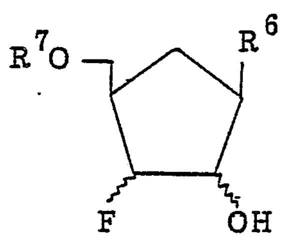

(I-3)

wherein $R^6$ is a residue of a nucleic acid base which may have a protecting group, an azido group or an amino group protected by a protecting group; and $R^7$ is a hydroxyl-protecting group, provided that the fluorine atom F and the hydroxyl group OH are located in a trans position to each other, which comprises fluorinating a 2,3-epoxy cyclopentane derivative of the formula:

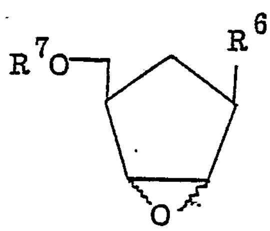

(III)

wherein $R^6$ and $R^7$ are as defined above, provided that the epoxy group is present at the α-or β-position, with a fluorinating agent.

17. A process for producing a 1β-substituted-2-hydroxy-3-fluoro-4β-hydroxymethyl cyclopentane derivative of the formula:

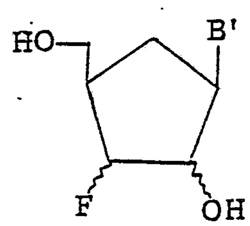

(I-4)

wherein B′ is a residue of a nucleic acid base, provided that the fluorine atom F and the hydroxyl group OH are present at a trans position to each other, which comprises converting $R^6$ of the product obtained in Claim 16 wherein $R^6$ is an azido group or an amino group protected by a protecting group, to an amino group simultaneously or subsequently removing the protecting group, and then converting the amino group to a residue of a nucleic acid base.

18. A process for producing a 1β-substituted-3(or 4)-fluoro-4β-hydroxymethyl cyclopentane derivative of the formula:

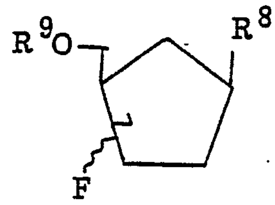

(I-5)

wherein $R^8$ is a residue of a nucleic acid base which may have a protecting group, an azido group or an amino group protected by a protecting group, and $R^9$ is a hydroxyl-protecting group, provided that the fluorine atom F is present at the 3α-, 3β-or 4α-position, which comprises converting Z of a fluorocyclopentane derivative of the formula:

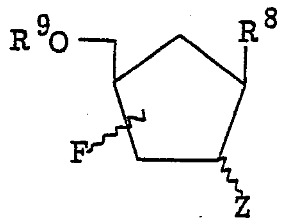

(III)

wherein $R^8$, $R^9$ and the position of the fluorine atom are as defined above, and Z is a hydroxyl group, an activated hydroxyl group or a bromine atom, to a hydrogen atom.

19. A process for producing a 1β-substituted-3(or 4)-fluoro-4β-hydroxymethyl cyclopentane derivative of the formula:

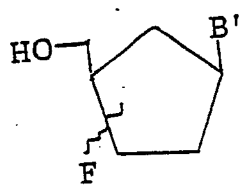

(I-6)

wherein B' is a residue of a nucleic acid base, provided that the fluorine atom F is present at the 3α-, 3β-or 4α-position, which comprises converting $R^8$ of the product obtained in the process of Claim 17 wherein $R^8$ is an azido group or an amino group protected by a protecting group, to an amino group, simultaneously or subsequently removing the protecting group, and then converting the amino group to a residue of a nucleic acid base.